# FASCICULE DE BREVET EUROPEEN

(11) **EP 0 991 763 B1**
(45) Date de publication et mention de la délivrance du brevet: **28.09.2005**
(21) Numéro de dépôt: 98917294.5
(22) Date de dépôt: 02.04.1998
(51) Int. Cl.: C12N 15/34, C12N 15/86, C07K 14/075, C12N 5/10, C12N 7/00, A61K 48/00

(54) **FIBRE ADENOVIRALE MODIFIEE ET ADENOVIRUS CIBLES**
MODIFIZIERTE ADENOVIRALE FIBER UND SIE ENTHALTENDE ADENOVIREN
MODIFIED ADENOVIRAL FIBER AND TARGET ADENOVIRUSES

(30) Priorité: 02.04.1997 FR 9703987; 17.04.1997 FR 9704747
(43) Date de publication de la demande: 12.04.2000
(73) Titulaire: TRANSGENE S.A., 67000 Strasbourg (FR); CENTRE NATIONAL DE LA RECHERCHE SCIENTIFIQUE, 75016 Paris Cédex (FR)
(72) Inventeur: LEGRAND, Valérie, F-67200 Strasbourg (FR); MEHTALI, Majid, F-67400 Illkirch Graffenstaden (FR); BOULANGER, Pierre, F-34000 Montpellier (FR)
(74) Mandataire: Warcoin, Jacques
(86) Numéro de dépôt international: PCT/FR1998/000668
(87) Numéro de publication internationale: WO 1998/044121

(56) Documents cités:
- WO-A-94/17832
- WO-A-95/05201
- WO-A-95/26412
- WO-A-96/26281
- J. GALL ET AL.: "Adenovirus type 5 and 7 capsid chimaera: Fiber replacement alters receptor tropism without affecting primary immune neutralization epitopes" JOURNAL OF VIROLOGY., vol. 70, no. 4, avril 1996, pages 2116-2123, XP002050655 AMERICAN SOCIETY FOR MICROBIOLOGY US
- A. MCCLELLAND ET AL: "Modification of the adenovirus fiber protein for targeted gene delivery" JOURNAL OF CELLULAR BIOCHEMISTRY - SUPPLEMENT, 26 mars 1995, page 411 XP000608380

## Description

La présente invention a pour objet une fibre adénovirale mutée dans les régions impliquées dans la reconnaissance et la liaison du récepteur cellulaire naturel des adénovirus. Elle concerne également les adénovirus recombinants portant à leur surface une telle fibre et un ligand qui leur confère une spécificité d'hôte modifiée ou ciblée envers un type cellulaire particulier, les cellules contenant ces adénovirus ainsi qu'une méthode pour préparer des particules virales infectieuses de ces derniers destinées à un usage thérapeutique. L'invention présente un intérêt tout particulier pour des perspectives de thérapie génique, notamment chez l'homme.

Grâce à leurs propriétés particulières, les adénovirus sont employés dans un nombre croissant d'applications en thérapie génique. Mis en évidence dans de nombreuses espèces animales, ils sont peu pathogènes, non-intégratifs et se répliquent aussi bien dans les cellules en division que quiescentes. De plus, ils présentent un large spectre d'hôte et sont capables d'infecter un très grand nombre de types cellulaires tels que les cellules épithéliales, endothéliales, les myocytes, les hépatocytes, les cellules nerveuses et les synoviocytes (Bramson et al., 1995, Curr. Op. Biotech. *6*, 590-595). Cependant, cette absence de spécificité d'infection pourrait constituer une limite à l'utilisation des adénovirus recombinants, d'une part, au niveau de la sécurité puisqu'il peut y avoir dissémination du gène recombinant dans l'organisme hôte et, d'autre part, au niveau de l'efficacité puisque le virus n'infecte pas spécifiquement le type cellulaire que l'on souhaite traiter.

D'une manière générale, le génome adénoviral est constitué d'une molécule d'ADN linéaire, bicaténaire et d'environ 36kb contenant les gènes codant pour les protéines virales et à ses extrémités deux répétitions inversées (désignées ITR pour Inverted Terminal Repeat) intervenant dans la réplication et la région d'encapsidation. Les gènes précoces sont répartis en 4 régions dispersées dans le génome adénoviral (E1 à E4 ; E pour early en anglais), comportant 6 unités transcriptionnelles munies de leurs propres promoteurs. Les gènes tardifs (L1 à L5 ; L pour late en anglais) recouvrent en partie les unités de transcription précoces et sont, pour la plupart, transcrits à partir du promoteur majeur tardif MLP (pour Major Late Promoter en anglais).

A titre indicatif, tous les adénovirus utilisés dans les protocoles de thérapie génique sont déficients pour la réplication par délétion d'au moins la région E1 et sont propagés dans une lignée cellulaire de complémentation, qui fournit *en trans* les fonctions virales délétées. On utilise couramment la lignée 293, établie à partir de cellules de rein embryonnaire humain, qui complémente efficacement la fonction E1 (Graham et al., 1977, J. Gen. Virol. *36*, 59-72). Des vecteurs de seconde génération ont récemment été proposés dans la littérature. Ils conservent les régions *en cis* nécessaires à la réplication du.virus dans la cellule infectée (ITRs et séquences d'encapsidation) et comportent des délétions internes importantes visant à supprimer l'essentiel des gènes viraux dont l'expression *in vivo* peut conduire à l'établissement de réponses inflammatoires ou immunitaires chez l'hôte. Les vecteurs adénoviraux et leur technique de préparation ont fait l'objet de nombreuses publications accessibles à l'homme du métier.

Le cycle infectieux des adénovirus se déroule en 2 étapes. La phase précoce précède l'initiation de la réplication et permet de produire les protéines précoces régulant la réplication et la transcription de l'ADN viral. La réplication du génome est suivie de la phase tardive au cours de laquelle sont synthétisées les protéines structurales qui constituent les particules virales. L'assemblage des nouveaux virions prend place dans le noyau. Dans un premier temps, les protéines virales s'assemblent de manière à former des capsides vides de structure icosaédrique dans lesquelles le génome est encapsidé. Les adénovirus libérés sont susceptibles d'infecter d'autres cellules permissives. A cet égard, la fibre et le penton base présents à la surface des capsides jouent un rôle critique dans l'attachement cellulaire des virions et leur internalisation.

L'adénovirus se lie à un récepteur cellulaire présent à la surface des cellules permissives par l'intermédiaire de la fibre trimérique (Philipson et al., 1968, J. Virol. *2,* 1064-1075 ; Defer et al., 1990, J. Virol. *64*, 3661-3673). La particule est ensuite internalisée par endocytose par la liaison du penton base aux intégrines cellulaires αᵥβ₃ et αᵥβ₅ (Mathias et al., 1994, J. Virol. *68*, 6811-6814). La capacité de la fibre soluble ou d'anticorps anti-fibre à inhiber l'infection démontre son rôle dans l'attachement cellulaire du virus.

La fibre est composée de 3 domaines (Chroboczek et al., 1995, Current Top. Microbiol. Immunol. *199*, 165-200) :
(1) En N-terminal, la queue très conservée d'un sérotype à l'autre, interagit avec le penton base et assure l'ancrage de la molécule dans la capside.
(2) La tige est une structure en bâtonnet composée d'un certain nombre de répétitions de feuillets β, ce nombre variant selon les sérotypes.
(3) Enfin, à l'extrémité distale de la tige, la tête est une structure globulaire sphérique qui contient les signaux de trimérisation (Hong et Engler, 1996, J. Virol. *70*, 7071-7078 ; Novelli et Boulanger, 1991, J. Biol. Chem. *266,* 9299-9303 ; Novelli et Boulanger, 1991, Virology *185*, 365-376). De plus, la plupart des données expérimentales montrent que le domaine de la tête est responsable de la liaison aux cellules permissives (Henry et al., 1994, J. Virol *68*, 5239-5246; Louis et al., 1994, J. Virol. *68*, 4104-4106).

Des adénovirus "ciblés" dont la fibre native est modifiée de manière à reconnaître un récepteur cellulaire différent ont déjà été proposés dans la littérature. Ainsi, WO94/10323 décrit des mutants de la fibre d'Ad5 dans lesquels une séquence codant pour un fragment d'anticorps (de type scFv) est insérée à la fin de l'une des 22 unités répétitives de la tige dans le but de modifier la spécificité d'infection à l'égard des cellules présentant l'antigène cible. US 5,543,328 décrit une fibre chimère Ad5 dans laquelle le domaine de la tête est remplacé par le facteur de nécrose des tumeurs (TNF) de manière à interagir avec le récepteur cellulaire du TNF. Dans une autre construction, la fibre native d'Ad5 est fusionnée à son extrémité C-terminale au peptide ApoE permettant une liaison au récepteur LDL (pour low density lipoprotein en anglais) présent à la surface des cellules hépatiques. WO95/26412 décrit une fibre modifiée par incorporation d'un ligand à l'extrémité C-terminale qui conserve ses capacités de triménsation. WO96/26281 décrit une fibre chimère obtenue par remplacement d'une partie de la fibre native et, en particulier de la tête, par la partie équivalente d'une fibre adénovirale d'un autre sérotype et, éventuellement, par insertion à l'extrémité C-terminale d'un peptide RGD spécifique de la vitronectine.

Gall et al., J. virol. Vol 70, 1996, p2116-2123, décrit la préparation d'un adénovirus chimérique sur lequel on procède à la substitution complète des fibres de l'adénovirus de type5 par des fibres d'adénovirus de type 7. Cette substitution complète (tête, tige et queue de la fibre) est destinée à modifier la spécificité de reconnaissance de la particule adénovirale. McClelland et al., J. Cell. Biochem, 1995, p411, décrit le remplacement de la tête de la fibre d'Ad5 (délimitée par les nucléotides 405-592) par une séquence capable de reconnaître un site de liaison différent de celui de la fibre d'Ad5. Suite à cette substitution de l'intégralité de la tête de la fibre, les auteurs constatent qu'il est possible de modifier la spécificité de reconnaissance de la fibre adénovirable.

Comme précédemment indiqué, la spécificité d'infection d'un adénovirus est déterminée par l'attachement de la fibre adénovirale à un récepteur cellulaire situé à la surface des cellules permissives. La demande de brevet français 97 01005 a mis en évidence le rôle des antigènes du complexe majeur d'histocompatibilité de classe I et des modules III de la fibronectine à titre respectivement de récepteur primaire et de co-facteur des adénovirus. Mais d'autres protéines peuvent intervenir. A cet égard, des travaux récents ont présumé l'utilisation du récepteur cellulaire des coxsachie virus par les adénovirus de types 2 et 5 pour pénétrer dans leurs cellules cibles (Bergelson et al., 1997, Science *275*, 1320-1323). Le problème que la présente invention se propose de résoudre est de modifier la région d'interaction de !a fibre adenovirale avec le(s) récepteur(s) cellulaire(s) afin d'altérer la spécificité d'hôte naturelle des adénovirus portant la fibre mutée. Pour faciliter la compréhension, on utilisera ci-après le terme «récepteur cellulaire» des adénovirus pour désigner le ou les polypeptides cellulaires intervenant directement ou non dans la liaison des adénovirus à leurs cellules cibles naturelles ou dans la pénétration au sein de ces dernières. Bien entendu, ledit récepteur peut être différent selon les sérotypes. L'addition d'un ligand permet de conférer un nouveau tropisme envers un ou plusieurs types cellulaires spécifiques ponant à leur surface une molécule cible reconnue par le ligand en question.

La présente invention constitue une amélioration de la technique antérieure puisqu'elle divulgue les régions de la fibre à muter pour inhiber ou empêcher la liaison au récepteur cellulaire naturel des adénovirus. On a maintenant substitué un ou plusieurs résidus de la région 443 à 462 de la tête de la fibre d'Ad5 et montré une inhibition de l'infectivité des adénovirus correspondants à l'égard des cellules normalement permissives. L'introduction du ligand GRP (pour gastrin releasing peptide en anglais) au sein de ces fibres devrait permettre de cibler l'infection vers les cellules exprimant le récepteur au GRP. Le but de la présente invention est de diminuer les quantités thérapeutiques d'adénovirus à utiliser et de cibler l'infection au niveau des cellules à traiter. Cette spécificité est indispensable lorsque l'on met en oeuvre un adénovirus exprimant un gène cytotoxique pour éviter la propagation de l'effet cytotoxique aux cellules saines. Les avantages procurés par la présente invention sont de réduire les risques de dissémination et les effets secondaires liés à la technologie adénovirale.

C'est pourquoi la présente invention a pour objet une fibre d'un adénovrus modifiée par mutation d'un ou plusieurs résidus de ladite fibre, caractérisée en ce que lesdits résidus sont dirigés vers le récepteur cellulaire naturel dudit adénovirus.

Le terme "fibre" est largement défini dans la partie introductive. La fibre de la présente invention peut dériver d'un adénovirus d'origine humaine, canine, aviaire, bovine, murine, ovine, porcine ou simienne ou encore être hybride et comprendre des fragments d'origines diverses. Concernant les adénovirus humains, on préfère utiliser ceux de sérotype C et, notamment, les adénovirus de type 2 ou 5 (Ad2 ou Ad5). On indique que la fibre d'Ad2 comporte 580 acides aminés (aa) dont la séquence est divulguée dans Herissé et al. (1981, Nucleic Acid Res. *9,* 4023-4042). Celle d'Ad5 présente 582 aa et sa séquence présentée à l'identificateur de séquence 1 (SEQ ID NO: 1) a été déterminée par Chroboczek et Jacrot (1987, Virology *161*, 549-554). Lorsque la fibre de là présente invention est originaire d'un adénovirus animal, on a de préférence recours aux adénovirus bovins et, en particulier, ceux de la souche BAV-3. Ces derniers ont fait l'objet de nombreuses études et la séquence de la fibre est divulguée dans la demande internationale WO95/16048. Bien entendu, la fibre de la présente invention peut présenter d'autres modifications par rapport à la séquence native, outre celles qui font l'objet de la présente invention.

Conformément aux buts poursuivis par la présente invention, la fibre selon l'invention est modifiée de manière à réduire ou abolir sa capacité de liaison au récepteur cellulaire naturel. Une telle propriété peut être vérifiée par l'étude de l'infectivité ou de la liaison cellulaire des virus correspondants en appliquant les techniques de l'art telles que celles détaillées ci-après. Selon un mode de réalisation avantageux, les propriétés de trimérisation et de liaison au penton-base ne sont pas affectées.

Au sens de la présente invention, le terme "mutation" désigne une délétion, une substitution ou encore une addition d'un ou plusieurs résidus ou une combinaison de ces possibilités. On préfère tout particulièrement le cas où les régions d'interaction avec le récepteur cellulaire naturel sont délétées en totalité ou en partie et remplacées notamment par un ligand spécifique d'une protéine de surface cellulaire autre que le récepteur naturel des adénovirus.

La structure crystallographique tridimensionnelle de la tête adénovirale a été déterminée par Xia et al. (1994, Structure 2, 1259-1270). Chaque monomère comporte 8 feuillets β antiparallèles désignés A à D et G à J et 6 boucles majeures de 8 à 55 résidus. Par exemple, la boucle CD relie le feuillet β C au feuillet β D. On indique que les feuillets mineurs E et F sont considérés comme faisant partie de la boucle DG située entre les feuillets D et G. A titre indicatif, le tableau 1 indique la localisation de ces structures dans la séquence en acides aminés de la fibre d'Ad5 telle que montrée à l'identificateur de séquence n° 1 (SEQ ID NO: 1), le +1 représentant le résidu Met initiateur. D'une manière générale, les feuillets forment une structure ordonnée et compacte alors que les boucles sont plus flexibles. Ces termes sont classiques dans le domaine de la biochimie des protéines et sont définis dans les ouvrages de base (voir par exemple Stryer, Biochemistry, 2ème édition, Chap 2, p 11 à 39, Ed Freeman et Compagny, San Francisco).

**Tableau 1**

| feuillet β | | boucle | |
|---|---|---|---|
| nomenclature | résidus | nomenclature | résidus |
| A | 400 à 403 | AB | 404 à 418 |
| B | 419 à 428 | - | - |
| C | 431 à 440 | CD | 441 à 453 |
| D | 454 à 461 | DG | 462 à 514 |
| G | 515 à 521 | GH | 522 à 528 |
| H | 529 à 536 | HI | 537 à 549 |
| I | 550 à 557 | IJ | 558 à 572 |
| J | 573 à 578 | | |

Les quatres feuillets β A, B, C et J constituent les feuillets V dirigés vers la particule virale. Les quatre autres (D, G, H et I) forment les feuillets R, supposés faire face au récepteur cellulaire. Les feuillets V semblent jouer un rôle important dans la trimérisation de la structure alors que les feuillets R seraient impliqués dans l'interaction avec le récepteur. Les résidus de la fibre d'Ad2, Ad3, Ad5, Ad7, Ad40, Ad41 et de l'adénovirus canin CAV formant ces différentes structures sont clairement indiqués dans la référence précédente.

Les modifications de la fibre adénovirale selon l'invention touchent plus particulièrement la partie qui comprend la boucle CD, le feuillet D et la partie proximale de la boucle DG (positions 441 à 478 de la fibre d'Ad2 et d'Ad5) et, plus particulièrement, la région s'étendant des résidus 443 à 462 pour ce qui est de l'Ad5 ou 451 à 466 dans le cas de l'Ad2. L'autre région cible pour les modifications est le feuillet H (aa 529 à 536 de l'Ad5 fibre et de la fibre d'Ad2). Une autre alternative consiste en la modification des feuillets mineurs E (aa 479-482 Ad5) et F (aa 485-486 Ad5).

Comme indiqué précédemment, on peut opérer par substitution d'un ou plusieurs acides aminés dans les régions exposées. On peut citer à ce titre les exemples suivants qui dérivent de la fibre d'Ad5 dans laquelle :
- le résidu glycine en position 443 est substitué par un acide aspartique,
- le résidu leucine en position 445 est substitué par une phénylalanine,
- le résidu glycine en position 450 est substitué par une asparagine,
- le résidu thréonine en position 451 est substitué par une lysine,
- le résidu valine en position 452 est substitué par une asparagine,
- le résidu alanine en position 455 est substitué par une phénylalanine,
- le résidu leucine en position 457 est substitué par une alanine ou une lysine, et/ou
- le résidu isoleucine en position 459 est substitué par une alanine.

Il est également possible d'introduire plusieurs substitutions au sein de la région ciblée de la fibre notamment au niveau des acides aminés formant un coude, de préférence de type αα (voir le Tableau 2 de Xia et al., 1994, *supra*). Pour illustrer, on peut citer les deux exemples suivants dans lesquels la fibre d'Ad5 est modifiée par substitution :
- du résidu glycine en position 443 par un acide aspartique,
- du résidu sérine en position 444 par une lysine, et
- du résidu alanine en position 446 par une thréonine
ou encore
- du résidu sérine en position 449 par un acide aspartique,
- du résidu glycine en position 450 par une lysine,
- du résidu thréonine en position 451 par une leucine, et
- du résidu valine en position 452 par une thréonine.

Bien entendu, les acides aminés de remplacement ne sont mentionnés qu'à titre indicatif et tout acide aminé peut convenir aux fins de la présente invention. On préfère cependant ne pas modifier de façon drastique la structure tridimensionnelle. De préférence, les acides aminés formant un coude seront remplacés par des résidus formant une structure similaire tels que ceux cités dans la référence Xia et al. déjà mentionnée.

La fibre de la présente invention peut également être modifiée par délétion. La région éliminée peut concerner tout ou partie du domaine exposé et, notamment de la boucle CD, du feuillet D, de la boucle DG et/ou des feuillets E et F. Concernant une fibre d'Ad5 selon l'invention, on peut citer plus particulièrement la délétion :
- de la région s'étendant de la sérine en position 454 à la phénylalanine en position 461,
- de la région s'étendant de la valine en position 441 à la glutamine en position 453,
- de la région s'étendant de la valine en position 441 à la phénylalanine en position 461, ou
- de la région s'étendant de l'asparagine en position 479 à la thréonine en position 486.

Il est également possible de générer d'autres mutants de substitution ou de délétion dans les autres feuillets ou boucles, comme par exemple les feuillets G, H et I et les boucles HI et DG.

Il est également possible lorsque l'une au moins des modifications est une délétion d'au moins 3 résidus consécutifs d'une boucle et/ou d'un feuillet, les résidus délétés peuvent être remplacés par des résidus d'une boucle et/ ou d'un feuillet équivalent dérivé d'une fibre d'un second adénovirus susceptible d'interagir avec un récepteur cellulaire différent de celui reconnu par le premier adénovirus. Le second adénovirus peut être d'une origine quelconque humaine ou animale. Ceci permet de maintenir la structure de la fibre selon l'invention tout en lui conférant une spécificité d'hôte correspondant à celle du second adénovirus. Comme indiqué dans Xia et al. (1994, *supra*), le récepteur cellulaire médiant l'infection des adénovirus de types 2 et 5 est différent de celui interagissant avec les adénovirus de types 3 et 7. Ainsi, une fibre d'Ad5 ou d'Ad2 délétée d'au moins 3 résidus consécutifs parmi ceux spécifiés ci-dessus peut-être substituée par les résidus issus d'une région équivalente de la fibre d'Ad3 ou d'Ad7 pour réduire sa capacité à lier le récepteur d'Ad5 et lui conférer une nouvelle spécificité envers le récepteur cellulaire d'Ad3 ou d'Ad7. A titre d'exemple non limitatif, on peut citer le remplacement des résidus LAPISGTVQSAHLIIRFD (positions 445 à 462) de la fibre d'Ad5 par les résidus VNTLFKNKNVSINVELYFD de la fibre d'Ad3 ou le remplacement des résidus PVTLTITL (position 529 à 536) de la fibre de l'Ad5 par les résidus PLEVTVML de la fibre de l'Ad3.

La présente invention concerne également une fibre d'un adénovirus présentant une capacité de liaison au récepteur cellulaire naturel substantiellement réduite et néanmoins capable de trimériser et de lier le penton base. Comme indiqué ci-avant, le récepteur cellulaire naturel est avantageusement choisi parmi le groupe constitué par les antigènes majeurs d'histocompatibilité de classe I, la fibronectine et le récepteur cellulaire des coxsachie virus (CAR) ou tout autre déterminant de surface cellulaire intervenant habituellement ou participant à l'infectivité des adénovirus.

Selon un mode de réalisation également avantageux, la fibre selon l'invention comprend en outre un ligand. Au sens de la présente invention, le terme ligand définit toute entité capable de reconnaître et lier, de préférence avec une forte affinité, une molécule de surface cellulaire différente du récepteur cellulaire naturel. Cette molécule peut être exprimée ou exposée à la surface de la cellule que l'on désire cibler (marqueur de surface cellulaire, récepteur, peptide antigénique présenté par les antigènes d'histocompatibilité...). Conformément aux buts poursuivis par la présente invention, un ligand peut être un anticorps, un peptide, une hormone, un polypeptide ou encore un sucre. Le terme anticorps comprend notamment les anticorps monoclonaux, les fragments d'anticorps (Fab) et les anticorps simple chaîne (scFv). Ces dénominations et abréviations sont conventionnelles dans le domaine de l'immunologie.

Dans le cadre de la présente invention, il peut être intéressant de cibler plus particulièrement une cellule tumorale, une cellule infectée, un type cellulaire particulier ou une catégorie de cellules portant un marqueur de surface spécifique. Par exemple, si la cellule hôte à cibler est une cellule infectée par le virus HIV (Human Immunodeficiency Virus), le ligand peut être un fragment d'anticorps contre la fusine, le récepteur CD4 ou contre une protéine virale exposée (glycoprotéine d'enveloppe) ou encore la partie de la protéine TAT du virus HIV s'étendant des résidus 37 à 72 ; (Fawell et al., 1994, Proc. Natl. Acad. Sci. USA *91*, 664-668). S'agissant d'une cellule tumorale, le choix se portera sur un ligand reconnaissant un antigène spécifique de tumeur (par exemple la protéine MUC-1 dans le cas du cancer du sein, certains épitopes des protéines E6 ou E7 du papilloma virus HPV) ou surexprimé (récepteur à l'IL-2 surexprimé dans certaines tumeurs lymphoïdes, peptide GRP pour Gastrin Releasing Peptide surexprimé dans les cellules de carcinome du poumon (Michaël et al., 1995 Gene Therapy 2, 660-668) et dans les tumeurs du pancréas de la prostate et de l'extomac). Si l'on désire cibler les lymphocytes T, on peut employer un ligand du récepteur de cellule T. Par ailleurs, la transferrine est un bon candidat pour un ciblage hépatique. D'une manière générale, les ligands qui peuvent être utilisés dans le contexte de l'invention sont largement décrits dans la littérature et peuvent être clonés par les techniques standards. Il est également possible de les synthétiser par voie chimique et les coupler à la fibre selon l'invention. A cet égard, le couplage de résidus galactosyl devrait conférer une spécificité hépatique en raison de l'interaction avec les récepteurs aux asialoglycoprotéines. Mais le mode de réalisation préféré consiste à insérer le ligand à l'extrémité C-terminale de la fibre selon l'invention ou en remplacement des résidus délétés lorsque l'une au moins des modifications est une délétion d'au moins 3 résidus consécutifs.

La présente invention a également trait à un fragment d'ADN codant pour une fibre selon l'invention ainsi qu'à un vecteur d'expression d'un tel fragment. Tout type de vecteur peut être employé à cet effet, qu'il soit d'origine plasmidique ou virale, intégratif ou non. De tels vecteurs sont disponibles commercialement ou décrits dans la littérature. De même, l'homme du métier est capable d'adapter les éléments de régulation nécessaires à l'expression du fragment d'ADN selon l'invention. En outre, il peut être associé à une ou plusieurs substances susceptibles d'améliorer l'efficacité transfectionnelle et/ou la stabilité du vecteur. Ces substances sont largement documentées dans la littérature accessible à l'homme de l'art (voir par exemple Felgner et al., 1989, Proc. West. Pharmacol. Soc. *32*, 115-121 ; Hodgson et Solaiman, 1996, Nature Biotechnology *14*, 339-342 ; Remy et al., 1994, Bioconjugate chemistry *5*, 647-654). A titre illustratif et non limitatif, il peut s'agir de polymères, de lipides notamment cationiques, de liposomes, de protéines nucléaires et de lipides neutres. Une combinaison envisageable est un vecteur associé à des lipides cationiques (DC-Chol, DOGS ...etc) et des lipides neutres (DOPE).

La présente invention concerne également un adénovirus dépourvu d'une fibre native fonctionnelle et qui comprend à sa surface une fibre selon l'invention. Celle-ci peut être exprimée par le génome adénoviral ou apportée *en trans* par une lignée cellulaire de complémentation, telle que celles définies ci-après. Il peut en outre comprendre un ligand tel que défini ci-dessus. De préférence, la spécificité de liaison d'un tel adénovirus à son récepteur cellulaire naturel est réduite de manière significative ou mieux abolie, du fait de la fibre modifiée qu'il porte. La perte de la spécificité naturelle peut être évaluée par des études d'attachement cellulaire réalisées en présence de virus marqués (par exemple à la ³H thymidine selon la technique de Roelvink et al., 1996, J. Virol. *70*, 7614-7621) ou par des études d'infectivité de cellules permissives ou exprimant la molécule de surface ciblée par le ligand (voir les exemples qui suivent).

Le ligand peut être couplé de manière chimique à l'adénovirus selon l'invention. Mais, on préfère la variante selon laquelle les séquences codant pour le ligand sont insérées au sein du génome adénoviral, en particulier, au sein des séquences codant pour la fibre modifiée selon l'invention, de préférence, en phase afin de préserver le cadre de lecture. L'insertion peut avoir lieu à un endroit quelconque. Néanmoins, le site d'insertion préféré est en amont du codon stop à l'extrémité C-terminale ou à la place des résidus délétés. Il est également envisageable d'introduire les séquences du ligand au sein d'autres séquences adénovirales, notamment celles codant pour une autre protéine de capside, comme l'hexon ou le penton.

Avantageusement, un adénovirus selon l'invention est recombinant et défectif pour la réplication, c'est à dire incapable de réplication autonome dans une cellule hôte. La déficience est obtenue par mutation ou délétion d'un ou plusieurs gènes viraux essentiels et, notamment, de tout ou partie de la région E1. Des délétions au sein de la région E3 peuvent être envisagées pour accroître les capacités de clonage. Cependant, il peut être avantageux de conserver les séquences codant pour la protéine gp19k (Gooding et Wood, 1990, Critical Reviews of Immunology *10*, 53-71) afin de moduler les réponses immunitaires de l'hôte. Bien entendu, le génome d'un adénovirus selon l'invention peut également comprendre des délétions ou mutations supplémentaires affectant d'autres régions, notamment les régions E2, E4 et/ou L1-L5 (voir par exemple la demande internationale WO94/28152 et Ensinger et al., 1972, J. Virol. *10*, 328-339 décrivant la mutation thermosensible du gène DBP de E2).

Selon un mode de réalisation préféré, un adénovirus selon l'invention est recombinant et comprend un ou plusieurs gène(s) d'intérêt placé(s) sous le contrôle des éléments nécessaires à son (leur) expression dans une cellule hôte. Le gène en question peut être d'une origine quelconque, génomique, ADNc (ADN complémentaire) ou hybride (minigène dépourvu d'un ou plusieurs introns). Il peut être obtenu par les techniques conventionnelles de biologie moléculaire ou par synthèse chimique. Il peut coder pour un ARN anti-sens, un ribozyme ou un ARNm qui sera ensuite traduit en polypeptide d'intérêt. Celui-ci peut être cytoplasmique, membranaire ou être secrété de la cellule hôte. Par ailleurs, il peut s'agir de tout ou partie d'un polypeptide tel que trouvé dans la nature, d'un polypeptide chimère provenant de la fusion de séquences d'origines diverses, ou d'un polypeptide muté par rapport à la séquence native présentant des propriétés biologiques améliorées et/ou modifiées.

Dans le cadre de la présente invention, il peut être avantageux d'utiliser les gènes codant pour les polypeptides suivants :
- cytokines ou lymphokines (interférons α, β et γ, interleukines et notamment l'IL-2, l'IL-6, l'IL-10 ou l'IL-12, facteurs nécrosant des tumeurs (TNF), facteurs stimulateurs de colonies (GM-CSF, C-CSF, M-CSF...) ;
- récepteurs cellulaires ou nucléaires, notamment ceux reconnus par des organismes pathogènes (virus, bactéries, ou parasites) et, de préférence, par le virus VIH ou leurs ligands (fas ligand);
- protéines impliquées dans une maladie génétique (facteur VII, facteur VIII, facteur IX, dystrophine ou minidystrophine, insuline, protéine CFTR (Cystic Fibrosis Transmembrane Conductance Regulator), hormones de croissance (hGH);
- enzymes (uréase, rénine, thrombine....) ;
- inhibiteurs d'enzymes (α1-antitrypsine, antithrombine III, inhibiteurs de protéases virales...) ;
- polypeptides à effet anti-tumoral capables d'inhiber au moins partiellement l'initiation ou la progression de tumeurs ou cancers (anticorps, inhibiteurs agissant au niveau de la division cellulaire ou des signaux de transduction, produits d'expression des gènes suppresseurs de tumeurs, par exemple p53 ou Rb, protéines stimulant le système immunitaire....) ;
- protéines du complexe majeur d'histocompatibilité des classes I ou II ou protéines régulatrices agissant sur l'expression des gènes correspondants ;
- polypeptides capables d'inhiber une infection virale, bactérienne ou parasitaire ou son développement (polypeptides antigéniques ayant des propriétés immunogènes, épitopes antigéniques, anticorps, variants trans-dominants susceptibles d'inhiber l'action d'une protéine native par compétition...) ;
- toxines (thymidine kinase de virus simplex de l'herpès 1 (TK-HSV-1), ricine, toxine cholérique, diphtérique.....) ou immunotoxines
- marqueurs (β-galactosidase, luciférase....),
- polypeptide ayant un effet sur l'apoptose (inducteur d'apoptose : Bax.... inhibiteur d'apoptose Bcl2, Bclx), agents cytostatiques (p21, p16, Rb...), les apolipoprotéines (apoE....), SOD, catalase, oxyde nitrique synthase (NOS) ; et
- facteurs de croissance (FGF pour Fibroblast growth Factor, VEGF pour Vascular Endothelial cell growth Factor....).

Il est à signaler que cette liste n'est pas limitative et que d'autres gènes peuvent également être employés.

Par ailleurs, un adénovirus selon l'invention peut, en outre, comprendre un gène de sélection permettant de sélectionner ou identifier les cellules infectées. On peut citer les gènes *néo* (codant pour la néomycine phosphotransférase) conférant une résistance à l'antibiotique G418, *dhfr* (Dihydrofolate Réductase), CAT (Chloramphenicol Acetyl transférase), *pac* (Puromycine Acétyl-Transferase) ou encore *gpt* (Xanthine Guanine Phosphoribosyl Transferase). D'une manière générale, les gènes de sélection sont connus de l'homme de l'art.

Par éléments nécessaires à l'expression d'un gène d'intérêt dans une cellule hôte, on entend l'ensemble des éléments permettant sa transcription en ARN et la traduction d'un ARNm en protéine. Parmi ceux-ci, le promoteur revêt une importance particulière. Dans le cadre de la présente invention, il peut dériver d'un gène quelconque d'origine eucaryote ou même virale et peut être constitutif ou régulable. Par ailleurs, il peut être modifié de manière à améliorer l'activité promotrice, supprimer une région inhibitrice de la transcription, rendre un promoteur constitutif régulable ou vice versa, introduire un site de restriction..... Alternativement, il peut s'agir du promoteur naturel du gène à exprimer. On peut mentionner, à titre d'exemples, les promoteurs viraux CMV (Cytomegalovirus), RSV (Rous Sarcoma Virus), du gène TK du virus HSV-1, précoce du virus SV40 (Simian Virus 40), adénoviral MLP ou encore les promoteurs eucaryotes des gènes PGK (Phospho Glycerate kinase) murin ou humain, MT (métallothionéine), α1-antitrypsine et albumine (foie-spécifique), immunoglobulines (lymphocyte-spécifique). On peut également employer un promoteur spécifique de tumeur (α fétoprotéine AFP, Ido et al., 1995, Cancer Res. *55*, 3105-3109 ; MUC-1 ; PSA pour prostate specific antigen, Lee et al., 1996, J. Biol. Chem. *271*, 4561-4565 ; et flt1 specifique des cellules endothéliales, Morishita et al., 1995, J. Biol. Chem. *270*, 27948-27953).

Bien entendu, un gène d'intérêt en usage dans la présente invention peut en outre comprendre des éléments additionnels nécessaires à l'expression (séquence intronique, séquence signal, séquence de localisation nucléaire, séquence terminatrice de la transcription, site d'initiation de la traduction de type IRES ou autre....) ou encore à sa maintenance dans la cellule hôte. De tels éléments sont connus de l'homme de l'art.

L'invention concerne également un procédé de préparation d'un adénovirus selon l'invention, selon lequel :
- on transfecte le génome dudit adénovirus dans une lignée cellulaire appropriée,
- on cultive ladite lignée cellulaire transfectée dans des conditions appropriées pour permettre la production dudit adénovirus, et
- on récupère ledit adénovirus dans la culture de ladite lignée cellulaire transfectée et, éventuellement, on purifie substantiellement ledit adénovirus.

Le choix de la lignée cellulaire dépend des fonctions déficientes de l'adénovirus selon l'invention et on utilisera une lignée de complémentation capable de fournir *en trans* la ou les fonction(s) défectueuse(s). La lignée 293 convient pour complémenter la fonction E1 (Graham et al., 1977, J. Gen. Virol. 36, 59-72). Pour une double déficience E1 et E2 ou E4, on peut employer une lignée parmi celles décrites dans la demande de brevet français 96 04413. On peut également mettre en oeuvre un virus auxilliaire pour complémenter l'adénovirus défectif selon l'invention dans une cellule hôte quelconque ou encore un système mixte utilisant cellule de complémentation et virus auxilliaire dans lequel les éléments sont dépendants les uns des autres. Les moyens de propagation d'un adénovirus défectif sont connus de l'homme de l'art qui peut se référer par exemple à Graham et Prevec (1991, Methods in Molecular Biology, vol 7, p 190-128 ; Ed E.J. Murey, The Human Press Inc.). Le génome adénoviral est de préférence reconstitué *in vitro* dans *Escherichia coli (E. coli)* par ligation ou encore recombinaison homologue (voir par exemple la demande française 94 14470). Les procédés de purification sont décrits dans l'état de la technique. On peut citer la technique de centrifugation sur gradient de densité.

La présente invention concerne également une lignée cellulaire comprenant soit sous forme intégrée dans le génome ou sous forme d'épisome un fragment d'ADN codant pour une fibre selon l'invention placé sous le contrôle des éléments permettant son expression. Ladite lignée peut en outre être capable de complémenter un adénovirus déficient pour une ou plusieurs fonctions sélectionnées parmi celles codées par les régions E1, E2, E4 et L1-L5. Elle dérive de préférence de la lignée 293. Une telle lignée peut être utile à la préparation d'un adénovirus dont le génome est dépourvu de tout ou partie des séquences codant pour la fibre (de manière à produire une fibre non fonctionnelle). La présente invention a également pour objet le procédé correspondant, selon lequel :
- on transfecte le génome dudit adénovirus dans une lignée cellulaire selon l'invention,
- on cultive ladite lignée cellulaire transfectée dans des conditions appropriées pour permettre la production dudit adénovirus, et
- on récupère ledit adénovirus dans la culture de ladite lignée cellulaire transfectée et, éventuellement, on purifie substantiellement ledit adénovirus.

La présente invention couvre également une cellule hôte infectée par un adénovirus selon l'invention ou susceptible d'être obtenu par un procédé selon l'invention. Il s'agit avantageusement d'une cellule de mammifère et, notamment, d'une cellule humaine. Elle peut être primaire ou tumorale et d'une origine quelconque, par exemple hématopoïétique (cellule souche totipotente, leucocyte, lymphocyte, monocyte ou macrophage ...), musculaire (cellule satellite, myocyte, myoblaste, cellule musculaire lisse), cardiaque, nasale, pulmonaire, trachéale, hépatique, épithéliale ou fibroblaste.

L'invention a également pour objet une composition pharmaceutique comprenant à titre d'agent thérapeutique ou prophylactique, une cellule hôte ou un adénovirus selon l'invention ou susceptible d'être obtenu par un procédé selon l'invention, en association avec un support acceptable d'un point de vue pharmaceutique. La composition selon l'invention est, en particulier, destinée au traitement préventif ou curatif de maladies telles que les maladies génétiques (hémophilie, mucoviscidose, diabète ou myopathie de Duchenne, de Becker...), les cancers, comme ceux induits par des oncogènes ou des virus, les maladies virales, comme l'hépatite B ou C et le SIDA (syndrome de l'immunodéficience acquise résultant de l'infection par le VIH), les maladies virales récurrentes, comme les infections virales provoquées par le virus de l'herpès et les maladies cardiovasculaires dont les resténoses.

Une composition pharmaceutique selon l'invention peut être fabriquée de manière conventionnelle. En particulier, on associe une quantité thérapeutiquement efficace de l'agent thérapeutique ou prophylactique à un support tel qu'un diluant. Une composition selon l'invention peut être administrée par voie locale, systémique ou par aérosol, en particulier par voie intragastrique, sous-cutanée, intracardiaque, intramusculaire, intraveineuse, intrapéritonéale, intratumorale, intrapulmonaire, intranasale ou intratrachéale. L'administration peut avoir lieu en dose unique ou répétée une ou plusieurs fois après un certain délai d'intervalle. La voie d'administration et le dosage appropriés varient en fonction de divers paramètres, par exemple, de l'individu ou de la maladie à traiter ou encore du ou des gène(s) d'intérêt à transférer. En particulier, les particules virales selon l'invention peuvent être formulées sous forme de doses comprises entre 10⁴ et 10¹⁴ ufp (unités formant des plages), avantageusement 10⁵ et 10¹³ ufp et, de préférence, 10⁶ et 10¹² ufp. La formulation peut également inclure un diluant, un adjuvant, un excipient acceptable d'un point de vue pharmaceutique de même qu'un agent de stabilisation, de préservation et/ou de solubilisation. Une formulation en solution saline, non aqueuse ou isotonique convient particulièrement à une administration injectable. Elle peut être présentée sous forme liquide ou sèche (par exemple lyophilisat...) ou tout autre forme gallénique couramment employée dans le domaine pharmaceutique.

Enfin, la présente invention est relative à l'usage thérapeutique ou prophylactique d'un adénovirus ou d'une cellule hôte selon l'invention ou d'un adénovirus susceptible d'être obtenu par un procédé selon l'invention, pour la préparation d'un médicament destiné au traitement du corps humain ou animal par thérapie génique. Selon une première possibilité, le médicament peut être administré directement *in vivo* (par exemple par injection intraveineuse, dans une tumeur accessible, dans les poumons par aérosol...). On peut également adopter l'approche *ex vivo* qui consiste à prélever des cellules du patient (cellules souches de la moëlle osseuse, lymphocytes du sang périphérique, cellules musculaires...), de les transfecter ou infecter *in vitro* selon les techniques de l'art et de les réadminister au patient.

L'invention s'étend également à une méthode de traitement selon laquelle on administre une quantité thérapeutiquement efficace d'un adénovirus ou d'une cellule hôte selon l'invention à un patient ayant besoin d'un tel traitement.

### EXEMPLES

Les exemples suivants n'illustrent qu'un mode de réalisation de la présente invention.

Les constructions décrites ci-dessous sont réalisées selon les techniques générales de génie génétique et de clonage moléculaire, détaillées dans Maniatis et al., (1989, Laboratory Manual, Cold Spring Harbor, Laboratory Press, Cold Spring Harbor, NY) ou selon les recommandations du fabricant lorsqu'on utilise un kit commercial. Les étapes de clonage mettant en oeuvre des plasmides bactériens sont réalisées de préférence dans la souche *E. coli* 5K (Hubacek et Glover, 1970, J. Mol. Biol. *50*, 111-127) ou BJ 5183 (Hanahan, 1983, J. Mol. Biol. *166,* 557-580). On utilise préférentiellement cette dernière souche pour les étapes de recombinaison homologue. La souche NM522 (Stratagène) convient à la propagation des vecteurs phagiques M13. Les techniques d'amplification par PCR sont connues de l'homme de l'art (voir par exemple PCR Protocols-A guide to methods and applications, 1990, edité par Innis, Gelfand, Sninshy et White, Academic Press Inc). S'agissant de la réparation des sites de restriction, la technique employée consiste en un remplissage des extrémités 5' protubérantes à l'aide du grand fragment de l'ADN polymérase I d'*E*. *coli* (Klenow). Les séquences nucléotidiques Ad5 sont celles utilisées dans la banque de donnée Genebank sous la référence M73260.

En ce qui concerne la biologie cellulaire, les cellules sont transfectées selon les techniques standards bien connues de l'homme du métier. On peut citer la technique au phosphate de calcium (Maniatis et al., *supra*), mais tout autre protocole peut également être employé, tel que la technique au DEAE dextran, l'électroporation, les méthodes basées sur les chocs osmotiques, la microinjection ou les méthodes basées sur l'emploi de lipides cationiques. Quant aux conditions de culture, elles sont classiques. Dans les exemples qui suivent, on a recours à la lignée humaine 293 (ATCC CRL1573) et aux lignées murines Swiss 3T3 (ATCC CCL92), NR6 (Wells et al., 1990, Science *247*: 962-964), NR6-hEGFR (Schneider et al., 1986, Proc. Natl. Acad. Sci. USA 83, 333-336), Daudi HLA- (ATCC CCL213) et Daudi HLA+ (Quillet et al., 1988, J. Immunol. 141, 17-20). On indique que la lignée Daudi est établie à partir d'un lymphome de Burkitt et est naturellement déficiente en l'expression de la β-2 microglobuline et, de ce fait, ne possède pas à sa surface les molécules HLA de classe I (Daudi HLA-). La lignée cellulaire E8.1 dérivée des Daudi a été générée par transfection d'un gène codant pour la β-2 microglobuline afin de restaurer l'expression de molécules HLA de classe I à leur surface (Daudi-HLA+ ; Quillet et al., 1988, J. Immunol. *141*, 17-20). Il est entendu que d'autres lignées cellulaires peuvent également être utilisées.

### EXEMPLE 1 : Construction d'un adénovirus présentant un tropisme d'hôte envers les cellules exprimant le récepteur du GRP (pour gastrin releasing peptide en anglais).

### A. Insertion des séquences codant pour le ligand GRP (fibre-GRP).

Le plasmide pTG6593 dérive du p poly II (Lathe et al., 1987, Gene *57*, 193-201) par introduction du gène complet codant pour la fibre d'Ad5 sous la forme d'un fragment *Eco*RI-*Sma*I (nucléotides (nt) 30049 à 33093). Le fragment *Hin*dIII-*Sma*I (nt 31994-33093) est isolé et cloné dans M13TG130 (Kieny et al., 1983, Gene 26, 91-99) digéré par ces mêmes enzymes, pour donner M13TG6526. Ce dernier est soumis à une mutagénèse dirigée à l'aide de l'oligonucléotide oTG7000 (SEQ ID NO: 2) (kit Sculptor, in vitro mutagenesis, Amersham) afin d'introduire un adaptateur codant pour un bras espaceur de 12 acides aminés de séquence PSASASASAPGS. Le vecteur muté ainsi obtenu, M13TG6527, est soumis à une seconde mutagénèse permettant d'introduire la séquence codant pour les 10 résidus du peptide GRP (GNHWAVGHLM ; Michael et al., 1995, Gene Ther. 2, 660-668). On utilise à cet effet l'oligonucléotide oTG7001 (SEQ ID NO: 3). Le fragment *Hin*dIII-*Sma*I est isolé du phage muté M13TG6528 et introduit par la technique de recombinaison homologue (Chartier et al., 1996, J. Virol. 70, 4805-4810) dans le plasmide pTG6590 portant le fragment de génome adénoviral Ad5 s'étendant des nt 27081 à 35935 et linéarisé par *Mun*I (nt 32825). Le fragment *Spe*I-*Sca*I (portant les nt 27082 à 35935 du génome Ad5 modifiés par introduction du bras espaceur et du peptide GRP) est isolé du vecteur précédent désigné pTG8599 puis est échangé contre le fragment équivalent de pTG6591 préalablement digéré par ces mêmes enzymes. A titre indicatif, pTG6591 comprend les séquences adénovirales sauvages des positions 21562 à 35935. On obtient pTG4600 dont on isole le fragment *Bst*EII (nt 24843 à 35233). Après recombinaison homologue avec le plasmide pTG3602 qui comprend le génome Ad5 (décrit plus en détail dans la demande internationale WO96/17070), on génère le vecteur pTG4601.

Une cassette permettant l'expression du gène LacZ est introduite à la place de la région adénovirale E1 par recombinaison homologue entre le plasmide pTG4601 linéarisé par *Cla*I et un fragment *Bsr*GI-*Pst*I comprenant le gène LacZ codant pour la β-galactosidase sous le contrôle du promoteur MLP d'Ad2 et le signal de polyadénylation du virus SV40. Ce fragment est isolé du vecteur pTG8526 contenant l'extrémité 5' de l'ADN génomique viral (nt 1 à 6241) dans lequel la région E1 (nt 459 à 3328) est remplacée par la cassette d'expression LacZ. Sa construction est à la portée de l'homme du métier. Le vecteur final est désigné pTG4628.

Les virus correspondants AdTG4601 et AdTG4628 sont obtenus par transfection des fragments adénoviraux libérés des séquences plasmidiques par digestion *Pac*I dans la lignée 293. A titre indicatif, AdTG4601 porte le génome Ad5 complet dans lequel le gène de la fibre comprend en son extrémité 3' un bras espaceur suivi du peptide GRP. Le virus recombinant AdTG4628 porte en outre la cassette d'expression du gène rapporteur LacZ sous le contrôle du promoteur adénoviral MLP.

### B. Etude du tropisme du virus portant la fibre-GRP.

La présence du peptide GRP au niveau de la fibre adénovirale permet de cibler les cellules exprimant à leur surface le récepteur au GRP. L'expression des messagers codant pour ce dernier est étudiée dans les cellules 293 et dans les cellules murines Swiss-3T3 (Zachary et al., 1985, Proc. Natl. Acad. Sci. USA 82, 7616-7620) par Northern-blot. On utilise à titre de sonde un mélange de 2 fragments d'ADN complémentaires à !a séquence codant pour le récepteur au GRP marqués par les techniques conventionnelles à l'isotope ³²P. A titre indicatif, les fragments sont produits par PCR réverse à partir des ARN cellulaires totaux à l'aide des oligonucléotides oTG10776 (SEQ ID NO: 4) et oTG10781(SEQ ID NO: 5) (Battey et al., 1991, Proc. Natl. Acad. Sci. USA 88, 395-399 ; Corjay et al., 1991, J. Biol. Chem. *266*, 18771-18779). L'intensité des ARNm détectés est beaucoup plus importante dans le cas des cellules Swiss-3T3 que dans les cellules 293, indiquant la surexpression du récepteur GRP par la lignée murine.

Des expériences de compétition sont réalisées sur les 2 types de cellules. Le compétiteur est constitué par la tête de la fibre d'Ad5 produite dans *E.coli* dont les propriétés de liaison au récepteur cellulaire adénoviral ont été montrées (Henry et al., 1994, J. Virol *68*, 5239-5246). Les cellules en monocouche sont préalablement incubées pendant 30 min en présence de PBS ou de concentrations croissantes de tête Ad5 recombinante (0,1 à 100 µg/ml) dans du milieu DMEM (Gibco BRL) complémenté avec du sérum de veau foetal 2% (FCS). Puis, le virus AdTG4628 dont la fibre contient le peptide GRP est ajouté à une multiplicité d'infection de 0,001 unité infectieuse/cellule pour 24h à 37°C. On utilise à titre de contrôle et selon les mêmes conditions expérimentales, le virus recombinant AdLacZ (Stratford-Perricaudet et al., 1992, J. Clin. Invest. *90*, 626-630) qui porte un gène de la fibre natif. Les cellules sont ensuite fixées et l'expression du gène LacZ évaluée (Sanes et al., 1986, EMBO J. *5*, 3133-3142). Le nombre de cellules bleues est représentatif de l'efficacité de l'infection virale. Une inhibition par compétiton se traduit par une réduction du nombre de cellules colorées par rapport à un témoin sans compétiteur (PBS).

L'addition de tête Ad5 recombinante à une concentration de 100 µg/ml inhibe fortement l'infection des cellules 293 par les virus AdLacZ et AdTG4628 (taux d'inhibition de 95 et 98%). Ceci suggère que la présence du compétiteur empêche l'interaction de la fibre adénovirale avec son récepteur cellulaire naturel. Par contre, les deux virus ont un comportement différent sur les cellules Swiss-3T3. L'infection du virus AdTG4628 en présence de 100 µg/ml de compétiteur n'est que partiellement inhibée alors que, dans les mêmes conditions expérimentales, celle du virus AdLacZ ayant la fibre native est totalement inhibée. Ces résultats suggèrent que l'infection des cellules Swiss-3T3 par l'AdTG4628 est en partie médiée par un récepteur indépendant, probablement le récepteur au GRP que ces cellules surexpriment. En conclusion, l'addition du ligand GRP à l'extrémité C-terminale de la fibre favorise l'infection des cellules exprimant le récepteur au GRP d'une manière indépendante de l'intéraction fibre-récepteur cellulaire naturel.

### EXEMPLE 2 : Construction d'un adénovirus présentant un tropisme d'hôte envers les cellules exprimant le récepteur à l'EGF (Epidermal Growth Factor en anglais).

Cet exemple décrit une fibre portant les séquences EGF à son extrémité C-terminale. Pour cela, on met en oeuvre les oligonucléotides oTG11065 (SEQ ID NO: 6) et oTG11066 (SEQ ID NO: 7) pour amplifier un fragment *Hin*dIII-*Xba*I à partir du plasmide M13TG6527. Les oligonucléotides oTG11067 (SEQ ID NO: 8) et oTG11068 (SEQ ID NO: 9) permettent de générer un fragment *Xho*I-*Sma*I (allant du codon stop jusqu'au nt 33093) à partir de M13TG6527. L'ADN complémentaire de l'EGF, obtenu de l'ATCC (#59957), est amplifié sous forme d'un fragment *Xho*I-*Xba*I à l'aide des oligonucléotides oTG 11069 (SEQ ID NO: 10) et oTG11070 (SEQ ID NO: 11). Les 3 fragments digérés par les enzymes adéquates sont ensuite religués pour donner un fragment *Hind*III-*Sma*I contenant l'EGF fusionné à l'extrémité C-terminale de la fibre. On applique la même procédure de recombinaison homologue que celle décrite à l'exemple 1 pour replacer ce fragment dans son contexte génomique.

Cependant, on peut simplifier les étapes de clonage en introduisant un site unique *Bst*BI dans la région ciblée par les techniques classiques de mutagénèse. On obtient pTG4609 et pTG4213 avec LacZ. La recombinaison homologue entre pTG4609 linéarisé par *Bst*BI et le fragment *Hin*dIII-*Sma*I précédant génère le plasmide pTG4225 portant la région E1 sauvage. Son équivalent portant la cassette d'expression LacZ pTG4226 est obtenu par recombinaison homologue avec le pTG4213 digéré par *Bst*BI. Les virus AdTG4225 et AdTG4226 peuvent être produits classiquement par transfection d'une lignée cellulaire appropriée par exemple surexprimant le récepteur de l'EGF.

Pour tester la spécificité d'infection de ces virus, on peut utiliser les cellules fibroblastiques murines NR6 et les cellules NR6-hEGFR exprimant le récepteur de l'EGF humain. Des compétitions avec la tête d'Ad5 recombinante ou avec l'EGF permettent d'évaluer l'intervention des récepteurs cellulaires naturels et EGF pour médier l'infection des virus.

### EXEMPLE 3 : Modifications de la tête de la fibre pour éliminer la liaison au récepteur cellulaire naturel.

La mutation de la région de la fibre adénovirale impliquée dans l'interaction avec le récepteur cellulaire naturel a été entreprise afin d'éliminer la capacité de la fibre à lier son récepteur naturel et l'addition d'un ligand permettra de modifier le tropisme des adénovirus correspondants.

Les séquences de la fibre Ad5 codant pour la région s'étendant des résidus 443 à 462 et 529 à 536 ont été soumises à diverses mutations. La délétion du feuillet D met en oeuvre l'oligonucléotide de mutagénèse oTG7414 (SEQ ID NO: 12) et la délétion de la boucle CD l'oligonucléotide oTGA (SEQ ID NO: 13). L'oligonucléotide oTGB (SEQ ID NO: 14) permet quant à lui la délétion de la boucle CD et du feuillet D. L'oligonucléotide OTG 7416 (SEQ ID NO: 38) permet la délétion du feuillet H. Tous ces oligonucléotides contiennent un site *Bam*HI permettant de détecter facilement les mutants et, également, d'insérer les séquences codant pour un ligand, par exemple le peptide EGF.

Une autre série de modifications consiste à remplacer ces régions délétées par les séquences équivalentes issues de la fibre d'Ad3 (D+CD5 en D+CD3 signifie que la région CD et D de la fibre Ad5 est remplacée par son équivalent d'Ad3). En effet, de nombreuses données montrent que l'Ad5 et l'Ad3 ne se lient pas au même récepteur, de sorte qu'une telle substitution devrait abolir l'infection médiée par le récepteur Ad5 et cibler les cellules portant le récepteur Ad3. Le remplacement de la boucle CD Ad5 par celle de l'Ad3 met en oeuvre oTG11135 (SEQ ID NO: 15), le remplacement du feuillet D de la fibre Ad5 par celui de la fibre Ad3 est effectué par l'oligonucléotide oTG10350 (SEQ ID NO: 16) et le remplacement du feuillet D et de la boucle CD de l'Ad5 par ceux de l'Ad3 est réalisé sur le mutant précédent à l'aide de oTG11136 (SEQ ID NO: 17). Le remplacement du feuillet H est réalisé à l'aide de l'oligonucléotide OTG 10352 (SEQ ID NO: 39).

On a également modifié cette région cible de la tête adénovirale par une série de mutations ponctuelles :
- remplacement du coude αα GSLA en coude αα DKLT: oTGC (SEQ ID NO: 18),
- remplacement du coude αα SGTV en coude αα DKLT : oTGD (SEQ ID NO: 19),
- G443 en D (G443D) : oTGE (SEQ ID NO: 20),
- L445 en F (L445F) : oTGF (SEQ ID NO: 21),
- G450 en N (G450N) : oTGG (SEQ ID NO: 22),
- T451 en K (T451K) : oTGH (SEQ ID NO: 23),
- V452 en N (V425N) : oTGI (SEQ ID NO: 24),
- A 455 en F (A455F) : oTGJ (SEQ ID NO: 25),
- L457 en K (L457K) : oTGK (SEQ ID NO: 26),
- 1459 en A (I459A) : oTG L (SEQ ID NO: 27).

Les oTGE à I introduisent des mutations dans la boucle CD de la fibre adénovirale sur des acides aminés qui sont non conservatifs entre l'Ad5 et l'Ad3 alors que les oTGJ à K concernent des acides aminés du feuillet D non engagés dans une liaison hydrogène stabilisant la structure.

Les mutagénèses peuvent être réalisées sur le vecteur M13TG6526 ou M13TG6528. Le premier porte le fragment *Hind*III-*Sma*I sauvage et le second ce même fragment modifié par l'insertion des séquences GRP. Les plasmides portant le génome adénoviral peuvent être reconstitués comme décrit auparavant pour les plasmides pTG4609 (E1 sauvage) et pTG4213 (LacZ à la place de la région E1). Les virus sont générés par transfection des cellules 293 ou bien de cellules surexprimant le récepteur liant le ligand concerné. De telles cellules peuvent être générées par transfection de l'ADN complémentaire correspondant. On utilise de préférence des cellules qui n'expriment pas naturellement le récepteur cellulaire naturel des adénovirus, par exemple la lignée Daudi (ATCC CCL213).

La viabilité des différents mutants est évaluée par transfection de cellules 293 et 293-Fb+ (cellules 293 transfectées par un vecteur d'expression de la fibre d'Ad5 sauvage). Cependant, l'efficacité de transfection est variable d'une expérience à l'autre, même en ce qui concerne un adénovirus portant une fibre sauvage ayant incorporée le peptide GRP à son extrémité C-terminale (AdFbGRP). Pour standardiser les résultats, les plages obtenues après transfection des cellules 293-Fb+ sont tout d'abord amplifiées sur cette même lignée et les virus générés sont titrés : à ce stade ils peuvent porter soit la fibre sauvage soit la fibre mutante ou les 2 types. Les cellules 293 sont alors infectées avec ces virus à une faible multiplicité d'infection ne permettant d'infecter qu'environ 10% des cellules (MOI d'environ 0,2 unité infectieuse/cellule) et on détermine l'étendue de l'infection virale à 7 jours post-infection en mesurant l'accumulation de l'ADN viral et le titre viral. La propagation de l'infection étant dépendante de la fibre mutée, les mutants intéressants sont ceux qui ne donnent pas lieu à une infection productive, montrant que la mutation altère la liaison au récepteur naturel. La capacité de propagation des adénovirus portant la fibre mutée est comparée à celle d'un adénovirus sauvage et d'un AdFbGRP.

Les résultats montrent que l'insertion du peptide GRP à l'extrémité de la fibre sauvage réduit légèrement la croissance du virus correspondant par rapport à un Ad sauvage, mais le facteur de multiplication est néanmoins de l'ordre de 1000. Les mutations V452N, D+CD5 en D+CD3 et L445F réduisent de manière significative (facteur 3 à 11) l'affinité de la tête de la fibre mutée pour le récepteur cellulaire naturel des adénovirus. Les mutations ΔD (délétion du feuillet D de la fibre Ad5), ΔCD+D (délétion de la boucle CD et du feuillet D), CD5 en CD3 (remplacement de la boucle CD d'un Ad5 par son équivalent d'un Ad3), G443D, A455F, L457K et I459A abolissent la propagation des virus correspondants dans les cellules 293 (facteur de multiplication inférieur à 1).

Puis on vérifie la capacité des virus mutants à pénétrer dans les cellules cibles par le biais du récepteur au GRP. Les mutants intéressants sont ceux qui présentent un facteur de multiplication significatif (supérieur à 1). A ce titre, on peut employer une lignée désignée 293-GRPR exprimant le MHC-I et le récepteur au GRP à des niveaux élevés. Elle est générée par transfection des cellules 293 par un plasmide d'expression eucaryotique portant l'ADNc du récepteur au GRP (Corjay et al., 1991, J. Biol. Chem. *266*, 18771-18779). La cassette d'expression est constituée par le promoteur CMV précoce (Boshart et al., 1985, Cell *41*, 521), les séquences d'épissage du gène β-globine, l'ADNc codant pour le récepteur au GRP et les séquences polyA du gène β-globine. La sélection des transformants est effectuée en présence d'hygromycine (350 µg/ml) et 50 clones sont sélectionnés, amplifiés et testés pour l'expression de l'ARNm codant pour le récepteur par la technique de Northern. Les clones les plus producteurs sont rassemblés et désignés 293-GRPR. L'expression de la protéine peut également être controlée par FACS à l'aide d'un peptide GRP conjugué à la biotine et à l'avidine-FITC suivi d'une détection à l'aide de fluorosceine.

### EXEMPLE 4 : Insertion du ligand dans une protéine de capside autre que la fibre en association avec une des modifications de la fibre précitées.

Cet exemple décrit l'insertion du ligand EGF dans la protéine de capside hexon. Bien entendu, il est préférable que l'adénovirus correspondant ait perdu sa capacité d'attachement au récepteur cellulaire naturel. Son génome peut par exemple inclure un gène de la fibre modifié (voir exemple 3) ou être dépourvu d'une partie au moins des séquences de la fibre.

On construit un plasmide de transfert pour la recombinaison homologue couvrant la région du génome d'Ad5 codant pour l'hexon (nt 15842-21700). Le fragment d'Ad5 *Hin*dIII-*Xho*I (nt 18836-24816) est cloné dans pBSK+ (Stratagène) digéré par ces mêmes enzymes pour donner le plasmide pTG4224. Les séquences codant pour le peptide EGF sont introduites dans la boucle hypervariable L1 de l'hexon par création de fragments chimériques par PCR : hexon (nt19043-19647)-*Xba*I-EGF-*Bsr*GI-hexon (nt 19699-20312). Le fragment nt 19043 à 19647 est obtenu par amplification PCR à partir du plasmide pTG3602 avec les oligonucléotides oTG11102 (SEQ ID NO: 28) et oTG11103 (SEQ ID NO: 29). Le fragment nt19699 à 20312 est amplifié à partir du même ADN avec les oligonucléotides oTG11104 (SEQ ID NO: 30) et oTG11105 (SEQ ID NO: 31). L'EGF est cloné à partir de l'ADNc à l'aide des oligonucléotides oTG11106 (SEQ ID NO: 32) et oTG11107 (SEQ ID NO: 33) permettant de mettre la séquence codante de l'EGF en phase avec l'hexon. Les produits PCR sont digérés par les enzymes adéquates puis religués. Le fragment chimérique peut alors être inséré par recombinaison homologue dans le plasmide pTG4224 linéarisé par *Nde*I (nt 19549), pour donner pTG4229. Les séquences codant pour l'hexon modifié peuvent être obtenues par digestion *Hin*dIII-*Xho*I et replacées dans leur contexte génomique par recombinaison homologue. On peut utiliser le vecteur pTG3602, pTG4607, pTG4629 linéarisé par *Sgf*I ou un vecteur portant le génome adénoviral délété des séquences de la fibre (comme pTG4607 décrit ci-dessous) ou exprimant une fibre modifiée conformément à l'exemple 3.

Le génome adénoviral incapable de produire une fibre native fonctionnelle est obtenu par une délétion touchant le codon initiateur mais ne s'étendant pas aux autres ORFs adénoviraux. On procède de la façon suivante: le fragment adénoviral en 5' de la délétion (nt 30564 à 31041) est amplifié par PCR à l'aide des amorces oTG7171 et oTG7275 (SEQ ID NO: 34 et 35). L'amplification du fragment en 3' (nt 31129 à 33099) met en oeuvre les amorces oTG7276 et oTG7049 (SEQ ID NO: 36 et 37). Les fragments PCR sont digérés par *Xho*I et mis en ligation avant d'être introduits par recombinaison homologue dans le vecteur pTG6591 linéarisé par *Nde*I, pour donner pTG4602. Puis le fragment *Bst*EII isolé de ce dernier est soumis à une recombinaison homologue avec ie vecteur pTG3602 digéré par *Spe*I. On obtient pTG4607. Le vecteur pTG4629 est équivalent à pTG4607, mais porte en outre la cassette d'expression *Lac*Z à la place de E1.

Les virus correspondants peuvent être obtenus après transfection de cellules 293, 293-Fb+ ou de cellules surexprimant le récepteur de l'EGF. L'étude de la spécificité d'infection pourra être réalisée comme décrit auparavant en utilisant l'EGF en tant que compétiteur.

### EXEMPLE 5 : Construction d'un mutant de la fibre délété des feuillets E et F.

On a également généré un mutant de délétion des feuillets EF du domaine de la tête de la fibre d'Ad5. Le plasmide pTG6593 est digéré par *Hin*dIII et *Sma*I et le fragment portant les séquences de la fibre est isolé et cloné dans le vecteur M13TG130 clivé par *Hin*dIII et *Sma*I. La mutation délétère met en oeuvre l'oligonucléotide indiqué dans la SEQ ID NO: 40. Le fragment *Hin*dIII-*Sma*I muté est recombiné dans *E. coli* avec pTG4609 linéarisé par *Bst*BI (Chartier et al., 1996, J. Virol. *70,* 4805-4810). Ce dernier contient le génome Ad5 complet comportant un site *Bst*BI en position 32940 en aval du codon stop de la fibre.

La capacité à trimériser de la fibre modifiée est testée sur gel NDS-PAGE (Novelli et Boulanger, J. of Biological Chemistry, 1991, *266*, 9299-9303) à partir de la protéine produite par voie recombinante dans les cellules d'insectes Sf9 à l'aide d'un baculovirus recombinant portant les séquences correspondantes placées sous le contrôle du promoteur polyhédrine. En parallèle, la viabilité (ou capacité à se propager) des virions portant la fibre mutée est déterminée par transfection des cellules 293 et 293Fb+. Enfin, la liaison de la fibre aux récepteurs cellulaires MHC-I et CAR peut être étudiée dans des expériences de compétition d'infection d'un adénovirus recombinant Ad5-Luc sur les cellules Daudi-HLA+ et CHO-CAR (Bergelson et al., 1997, Science *275*, 1320-1323). A titre indicatif, le virus Ad5Luc est un adénovirus compétent pour la réplication qui contient le gène luciférase placé sous le contrôle du promoteur précoce du virus SV40 (Virus simien 40) inséré dans la région E3 du génome adénoviral (Mittal et al., 1993, Virus Research *28*, 67-90).

La fibre ΔEF s'accumule sous forme de trimères, peut se propager dans les cellules 293 et 293Fb+ et n'est pas un compétiteur pour l'infection des cellules Daudi-HLA+ par l'Ad5Luc. Elle est capable d'inhiber partiellement l'infection des cellules CHO-CAR, mais moins efficacement que la fibre sauvage. Ceci suppose que les feuillets E et F sont importants pour l'attachement de l'Ad5 au récepteur MHC-I alors qu'il joue un rôle plus mineur, peut être de stabilisation dans la liaison au CAR. L'insertion d'un nouveau ligand devrait permettre de rediriger l'infectivité vers les cellules portant le récepteur reconnu par le ligand.

## Revendications

1. Fibre d'un adénovirus modifiée par mutation de substitution d'un ou plusieurs résidus de ladite fibre, **caractérisée en ce que** lesdits résidus sont dirigés vers le récepteur cellulaire naturel dudit adénovirus et compris dans la partie de ladite fibre qui comprend la boucle CD, le feuillet D et la boucle DG, ladite mutation ayant pour effet de réduire ou abolir la capacité de liaison de ladite fibre modifiée au récepteur cellulaire naturel.

2. Fibre d'un adénovirus selon la revendication 1, **caractérisée en ce qu'**elle est modifiée par mutation de substitution d'un ou plusieurs résidus dans la partie de ladite fibre qui comprend la boucle CD, le feuillet D et la partie proximale de la boucle DG.

3. Fibre d'un adénovirus selon la revendication 1 ou 2, **caractérisée en ce que** ledit adénovirus est l'adénovirus de type 5 (Ad5) et que ladite fibre comprend tout ou partie de la séquence telle que montrée à l'identificateur de séquence n° 1 (SEQ ID NO: 1) et qu'elle est modifiée par mutation de substitution d'un ou plusieurs résidus de la région comprise entre les résidus 441 à 478 de la SEQ ID NO: 1 et, de préférence, 443 à 462.

4. Fibre d'un adénovirus selon la revendication 1, **caractérisée en ce qu'**elle est modifiée par mutation de substitution d'un ou plusieurs résidus compris dans les feuillets E et F de ladite fibre.

5. Fibre d'un adénovirus selon la revendication 4, **caractérisée en ce que** ledit adénovirus est l'adénovirus 5 (Ad5) et que ladite fibre comprend tout ou partie de la séquence telle que montrée à l'identificateur de séquence n° 1 (SEQ ID NO : 1) et qu'elle est modifiée par mutation de substitution d'un ou plusieurs résidus de la région comprise entre les résidus 479 à 486 de la SEQ ID NO: 1.

6. Fibre d'un adénovirus Ad5 selon la revendication 3, **caractérisée en ce qu'**elle est modifiée par substitution :
- du résidu glycine en position 443 par un résidu acide aspartique,
- du résidu leucine en position 445 par un résidu phénylalanine,
- du résidu valine en position 452 par un résidu asparagine,
- du résidu alanine en position 455 par un résidu phénylalanine,
- du résidu leucine en position 457 par un résidu alanine ou lysine, ou
- du résidu isoleucine en position 459 par un résidu alanine.

7. Fibre d'un adénovirus selon la revendication 1 ou 2, **caractérisée en ce que** ledit adénovirus est l'adénovirus de type 2 (Ad2) et que ladite fibre est modifiée par mutation de substitution d'un ou plusieurs résidus de la région comprise entre les résidus 441 à 478 et, de préférence, 451 à 466 de ladite fibre.

8. Fibre d'un adénovirus selon la revendication 1, **caractérisée en ce que** ledit adénovirus est l'adénovirus de type 2 (Ad2) et que ladite fibre est modifiée par mutation de substitution d'un ou plusieurs résidus de la région comprise entre les résidus 479 à 486 de ladite fibre.

9. Fibre d'un adénovirus selon l'une des revendications 1 à 8 **caractérisée en ce qu'**elle présente une capacité de liaison au récepteur cellulaire naturel substantiellement réduite comparé à une fibre non modifiée et qu'elle est capable de trimériser.

10. Fibre d'un adénovirus selon l'une des revendications 1 à 9, **caractérisée en ce qu'**elle comprend en outre un ligand capable de reconnaître une molécule de surface cellulaire différente du récepteur cellulaire naturel dudit adénovirus.

11. Fibre d'un adénovirus selon la revendication 10, **caractérisée en ce que** le ligand est sélectionné parmi le groupe constitué par un anticorps, un peptide, une hormone, un polypeptide ou encore un sucre.

12. Fibre d'un adénovirus selon la revendication 10 ou 11, **caractérisée en ce que** le ligand est inséré à l'extrémité C-terminale de la fibre.

13. Fragment d'ADN ou vecteur d'expression codant pour une fibre d'un adénovirus selon l'une des revendications 1 à 12.

14. Lignée cellulaire **caractérisée en ce qu'**elle comprend soit sous forme intégrée dans le génome ou sous forme d'épisome un fragment d'ADN selon la revendication 13 placé sous le contrôle des éléments permettant son expression dans ladite lignée cellulaire.

15. Lignée cellulaire selon la revendication 14, **caractérisée en ce** en ce qu'elle est en outre capable de complémenter un adénovirus déficient pour une ou plusieurs fonctions sélectionnées parmi les fonctions codées par les régions E1, E2, E4 et L1-L5.

16. Lignée cellulaire selon la revendication 14 ou 15, **caractérisée en ce qu'**elle dérive de la lignée 293.

17. Adénovirus **caractérisé en ce qu'**il est dépourvu d'une fibre native fonctionnelle et qu'il comprend une fibre selon l'une des revendications 1 à 12, ladite fibre selon l'une des revendications 1 à 12 étant codée par le génome dudit adénovirus ou fournie *en trans* par une lignée cellulaire selon l'une des revendications 14 à 16.

18. Adénovirus **caractérisé en ce qu'**il est dépourvu d'une fibre fonctionnelle et qu'il comprend une fibre selon l'une des revendications 1 à 12 et un ligand capable de reconnaître une molécule de surface cellulaire différente du récepteur cellulaire naturel dudit adénovirus.

19. Adénovirus selon la revendication 18, **caractérisé en ce que** le ligand est sélectionné parmi le groupe constitué par un anticorps, un peptide, une hormone, un polypeptide ou encore un sucre.

20. Adénovirus selon l'une des revendications 18 et 19, **caractérisé en ce que** le ligand est inséré à l'extrémité C-terminale de la fibre.

21. Adénovirus selon l'une des revendications 17 à 19, **caractérisé en ce que** le ligand est inséré dans une protéine de capside autre que la fibre, notamment l'hexon ou le penton.

22. Adénovirus selon l'une des revendications 17 à 21, **caractérisé en ce qu'**il s'agit d'un adénovirus recombinant défectif pour la réplication.

23. Adénovirus selon la revendication 22, **caractérisé en ce qu'**il est délété de tout ou partie de la région E1 et, optionnellement, de tout ou partie de la région E3.

24. Adénovirus selon la revendication 23, **caractérisé en ce qu'**il est en outre délété de tout ou partie de la région E2, E4 et/ou LI-L5.

25. Adénovirus selon l'une des revendications 22 à 24, **caractérisé en ce qu'**il comprend un gène d'intérêt sélectionné parmi les gènes codant pour une cytokine, un récepteur cellulaire ou nucléaire, un ligand, un facteur de coagulation, la protéine CFTR, l'insuline, la dystrophine, une hormone de croissance, une enzyme, un inhibiteur d'enzyme, un polypeptide à effet anti-tumoral, un polypeptide capable d'inhiber une infection bactérienne, parasitaire ou virale et, notamment le VIH, un anticorps, une toxine, une immunotoxine et un marqueur.

26. Procédé pour produire un adénovirus selon l'une des revendications 17 à 25, **caractérisé en ce que** :
- on transfecte le génome dudit adénovirus dans une lignée cellulaire appropriée,
- on cultive ladite lignée cellulaire transfectée dans des conditions appropriées pour permettre la production dudit adénovirus, et
- on récupère ledit adénovirus de ladite culture de ladite lignée cellulaire transfectée et, éventuellement, on purifie substantiellement ledit adénovirus.

27. Procédé pour produire un adénovirus dont le génome est dépourvu de tout ou partie des séquences codant pour une fibre, **caractérisé en ce que** :
- on transfecte le génome dudit adénovirus dans une lignée cellulaire selon l'une des revendications 14 à 16,
- on cultive ladite lignée cellulaire transfectée dans des conditions appropriées pour permettre la production dudit adénovirus, et
- on récupère ledit adénovirus dans la culture de ladite lignée cellulaire transfectée et, éventuellement, on purifie substantiellement ledit adénovirus.

28. Cellule hôte comprenant un adénovirus selon l'une des revendications 17 à 25 ou obtenu par un procédé selon la revendication 26 ou 27.

29. Composition pharmaceutique comprenant un adénovirus selon l'une des revendications 17 à 25 ou obtenu par un procédé selon la revendication 26 ou 27 ou une cellule hôte selon la revendication 28 en association avec un support acceptable d'un point de vue pharmaceutique.

30. Utilisation thérapeutique ou prophylactique d'un adénovirus selon l'une des revendications 17 à 25 ou obtenu par un procédé selon la revendication 26 ou 27 ou d'une cellule hôte selon la revendication 28, pour la préparation d'un médicament destiné au traitement du corps humain ou animal par thérapie génique.

## Patentansprüche

1. Faser eines Adenovirus, welche durch Substitutionsmutation von einem oder mehreren Resten der Faser modifiziert ist, **dadurch gekennzeichnet, dass** diese Reste in Richtung des natürlichen zellulären Rezeptors des Adenovirus gerichtet und in dem Abschnitt der Faser, der die CD-Schleife, das D-Faltblatt und die DG-Schleife umfasst, enthalten sind, wobei die Mutation zur Wirkung hat, die Bindungsfähigkeit der modifizierten Faser an den natürlichen zellulären Rezeptor zu verringern oder zu beseitigen.

2. Faser eines Adenovirus nach Anspruch 1, **dadurch gekennzeichnet, dass** sie durch Substitutionsmutation von einem oder mehreren Resten in dem Abschnitt der Faser, welcher die CD-Schleife, das D-Faltblatt und den proximalen Teil der DG-Schleife umfasst, modifiziert ist.

3. Faser eines Adenovirus nach Anspruch 1 oder 2, **dadurch gekennzeichnet, dass** das Adenovirus das Adenovirus vom Typ 5 (Ad5) ist und dass die Faser die Gesamtheit oder einen Teil der Sequenz, wie sie in der Sequenzbezeichnung Nr. 1 (SEQ ID NO: 1) gezeigt ist, umfasst und dass sie durch Substitutionsmutation von einem oder mehreren Resten aus der Region zwischen den Resten 441 bis 478 der SEQ ID NO:1 und vorzugsweise 443 bis 462 modifiziert ist.

4. Faser eines Adenovirus nach Anspruch 1, **dadurch gekennzeichnet, dass** sie durch Substitutionsmutation von einem oder mehreren Resten, die in den Faltblättern E und F der Faser enthalten sind, modifiziert ist.

5. Faser eines Adenovirus nach Anspruch 4, **dadurch gekennzeichnet, dass** das Adenovirus das Adenovirus 5 (Ad5) ist und dass die Faser die Gesamtheit oder einen Teil der Sequenz, wie sie in der Sequenzbeschreibung Nr. 1 (SEQ ID NO: 1) gezeigt ist, umfasst und dass sie durch Substitutionsmutation von einem oder mehreren Resten aus der Region zwischen den Resten 479 bis 486 der SEQ ID NO: 1 modifiziert ist.

6. Faser eines Ad5-Adenovirus nach Anspruch 3, **dadurch gekennzeichnet, dass** sie modifiziert ist durch Substitution:
- des Glycinrests an Position 443 durch einen Asparaginsäurerest,
- des Leucinrests an Position 445 durch einen Phenylalaninrest,
- des Valinrests an Position 452 durch einen Asparaginrest,
- des Alaninrests an Position 455 durch einen Phenylalaninrest,
- des Leucinrests an Position 457 durch einen Alanin- oder Lysinrest oder
- des Isoleucinrests an Position 459 durch einen Alaninrest.

7. Faser eines Adenovirus nach Anspruch 1 oder 2, **dadurch gekennzeichnet, dass** das Adenovirus das Adenovirus vom Typ 2 (Ad2) ist und dass die Faser durch Substitutionsmutation von einem oder mehreren Resten aus der Region zwischen den Resten 441 bis 478 und vorzugsweise 451 bis 466 der Faser modifiziert ist.

8. Faser eines Adenovirus nach Anspruch 1, **dadurch gekennzeichnet, dass** das Adenovirus das Adenovirus vom Typ 2 (Ad2) ist und dass die Faser durch Substitutionsmutation von einem oder mehreren Resten aus der Region zwischen den Resten 479 bis 486 der Faser modifiziert ist.

9. Faser eines Adenovirus nach einem der Ansprüche 1 bis 8, **dadurch gekennzeichnet, dass** sie eine substantiell verringerte Bindungsfähigkeit an den natürlichen zellulären Rezeptor verglichen mit einer nicht-modifizierten Faser aufweist und dass sie zur Trimerisierung in der Lage ist.

10. Faser eines Adenovirus nach einem der Ansprüche 1 bis 9, **dadurch gekennzeichnet, dass** sie außerdem einen Liganden umfasst, der in der Lage ist, ein von dem natürlichen zellulären Rezeptor des Adenovirus verschiedenes zelluläres Oberflächenmolekül zu erkennen.

11. Faser eines Adenovirus nach Anspruch 10, **dadurch gekennzeichnet, dass** der Ligand aus der Gruppe, welche aus einem Antikörper, einem Peptid, einem Hormon, einem Polypeptid oder ferner einem Zucker gebildet wird, ausgewählt wird.

12. Faser eines Adenovirus nach Anspruch 10 oder 11, **dadurch gekennzeichnet, dass** der Ligand an dem C-terminalen Ende der Faser inseriert ist.

13. DNA-Fragment oder Expressionsvektor, welches bzw. welcher eine Faser eines Adenovirus nach einem der Ansprüche 1 bis 12 kodiert.

14. Zelllinie, **dadurch gekennzeichnet, dass** sie entweder in in das Genom integrierter Form oder in Form eines Episoms ein DNA-Fragment nach Anspruch 13 umfasst, welches unter die Kontrolle der Elemente, die dessen Expression in der Zelllinie erlauben, gestellt ist.

15. Zelllinie nach Anspruch 14, **dadurch gekennzeichnet, dass** sie außerdem in der Lage ist, ein Adenovirus zu komplementieren, welches hinsichtlich einer oder mehrerer Funktionen, ausgewählt aus den durch die Regionen E1, E2, E4 und L1-L5 kodierten Funktionen, defizient ist.

16. Zelllinie nach Anspruch 14 oder 15, **dadurch gekennzeichnet, dass** sie von der Linie 293 abgeleitet ist.

17. Adenovirus, **dadurch gekennzeichnet, dass** es keine funktionsfähige native Faser aufweist und dass es eine Faser nach einem der Ansprüche 1 bis 12 umfasst, wobei die Faser nach einem der Ansprüche 1 bis 12 durch das Genom des Adenovirus kodiert wird oder *in trans* durch eine Zelllinie nach einem der Ansprüche 14 bis 16 bereitgestellt wird.

18. Adenovirus, **dadurch gekennzeichnet, dass** es keine funktionsfähige Faser aufweist und dass es eine Faser nach einem der Ansprüche 1 bis 12 und einen Liganden, welcher in der Lage ist, ein von dem natürlichen zellulären Rezeptor des Adenovirus verschiedenes zelluläres Oberflächenmolekül zu erkennen, umfasst.

19. Adenovirus nach Anspruch 18, **dadurch gekennzeichnet, dass** der Ligand aus der Gruppe, welche aus einem Antikörper, einem Peptid, einem Hormon, einem Polypeptid oder ferner einem Zucker gebildet wird, ausgewählt wird.

20. Adenovirus nach einem der Ansprüche 18 und 19, **dadurch gekennzeichnet, dass** der Ligand an dem C-terminalen Ende der Faser inseriert ist.

21. Adenovirus nach einem der Ansprüche 17 bis 19, **dadurch gekennzeichnet, dass** der Ligand in ein anderes Capsidprotein als die Faser, insbesondere das Hexon oder das Penton inseriert ist.

22. Adenovirus nach einem der Ansprüche 17 bis 21, **dadurch gekennzeichnet, dass** es sich um ein replikationsdefektes rekombinantes Adenovirus handelt.

23. Adenovirus nach Anspruch 22, **dadurch gekennzeichnet, dass** aus diesem die Gesamtheit oder ein Teil der E1-Region und gegebenenfalls die Gesamtheit oder ein Teil der E3-Region deletiert ist.

24. Adenovirus nach Anspruch 23, **dadurch gekennzeichnet, dass** aus diesem außerdem die Gesamtheit oder ein Teil der Region E2, E4 und/oder L1-L5 deletiert ist.

25. Adenovirus nach einem der Ansprüche 22 bis 24, **dadurch gekennzeichnet, dass** es ein Gen von Interesse umfasst, welches ausgewählt wird aus den Genen, die ein Zytokin, einen zellulären oder nukleären Rezeptor, einen Liganden, einen Gerinnungsfaktor, das CFTR-Protein, Insulin, Dystrophin, ein Wachstumshormon, ein Enzym, einen Enzyminhibitor, ein Polypeptid mit Antitumorwirkung, ein Polypeptid, das in der Lage ist, eine bakterielle, durch Parasiten verursachte oder virale und insbesondere durch HIV verursachte Infektion zu hemmen, einen Antikörper, ein Toxin, ein Immuntoxin und einen Marker kodieren.

26. Verfahren zur Produktion eines Adenovirus nach einem der Ansprüche 17 bis 25, **dadurch gekennzeichnet, dass**:
- man eine geeignete Zelllinie mit dem Genom des Adenovirus transfiziert,
- man die transfizierte Zelllinie unter Bedingungen, die geeignet sind, um die Produktion des Adenovirus zu ermöglichen, kultiviert und
- man das Adenovirus aus der Kultur der transfizierten Zelllinie gewinnt und man gegebenenfalls das Adenovirus im Wesentlichen reinigt.

27. Verfahren zur Produktion eines Adenovirus, in dessen Genom die Gesamtheit oder ein Teil der eine Faser kodierenden Sequenzen fehlt, **dadurch gekennzeichnet, dass**:
- man eine Zelllinie nach einem der Ansprüche 14 bis 16 mit dem Genom des Adenovirus transfiziert,
- man die transfizierte Zelllinie unter Bedingungen, die geeignet sind, um die Produktion des Adenovirus zu ermöglichen, kultiviert und
- man das Adenovirus in der Kultur der transfizierten Zelllinie gewinnt und man gegebenenfalls das Adenovirus im Wesentlichen reinigt.

28. Wirtszelle, welche ein Adenovirus nach einem der Ansprüche 17 bis 25, oder welches durch ein Verfahren nach Anspruch 26 oder 27 erhalten wird, umfasst.

29. Pharmazeutische Zusammensetzung, welche ein Adenovirus nach einem der Ansprüche 17 bis 25, oder welches durch ein Verfahren nach Anspruch 26 oder 27 erhalten wird, oder eine Wirtszelle nach Anspruch 28 in Kombination mit einem aus pharmazeutischer Sicht annehmbaren Träger umfasst.

30. Pharmazeutische oder prophylaktische Verwendung eines Adenovirus nach einem der Ansprüche 17 bis 25, oder welches durch ein Verfahren nach Anspruch 26 oder 27 erhalten wird, oder einer Wirtszelle nach Anspruch 28 für die Herstellung eines Arzneimittels, welches für die Behandlung des menschlichen oder tierischen Körpers durch Gentherapie bestimmt ist.

## Claims

1. Adenovirus fibre modified by mutation consisting of substitution of one or more residues of said fibre, **characterized in that** said residues are directed towards the natural cellular receptor of said adenovirus and are included in the portion of said fibre which comprises the CD loop, the sheet D and the DG loop, said mutation having the effect of reducing or abolishing the capacity of said modified fibre for binding to the natural cellular receptor.

2. Adenovirus fibre according to Claim 1, **characterized in that** it is modified by mutation consisting of substitution of one or more residues in the portion of said fibre which comprises the CD loop, the sheet D and the proximal portion of the DG loop.

3. Adenovirus fibre according to Claim 1 or 2, **characterized in that** said adenovirus is the type 5 adenovirus (Ad5) and **in that** said fibre comprises all or part of the sequence as shown in the sequence identifier No. 1 (SEQ ID NO: 1) and **in that** it is modified by mutation consisting of substitution of one or more residues of the region between residues 441 to 478 of SEQ ID NO: 1 and, preferably, 443 to 462.

4. Adenovirus fibre according to Claim 1, **characterized in that** it is modified by mutation consisting of substitution of one or more residues included in the sheets E and F of said fibre.

5. Adenovirus fibre according to Claim 4, **characterized in that** said adenovirus is the type 5 adenovirus (Ad5) and **in that** said fibre comprises all or part of the sequence as shown in the sequence identifier No. 1 (SEQ ID NO: 1) and **in that** it is modified by mutation consisting of substitution of one or more residues of the region between residues 479 to 486 of SEQ ID NO: 1.

6. Ad5 adenovirus fibre according to Claim 3, **characterized in that** it is modified by substitution:
- of the glycine residue at position 443 by an aspartic acid residue,
- of the leucine residue at position 445 by a phenylalanine residue,
- of the valine residue at position 452 by an asparagine residue,
- of the alanine residue at position 455 by a phenylalanine residue,
- of the leucine residue at position 457 by an alanine or lysine residue, and/or
- of the isoleucine residue at position 459 by an alanine residue.

7. Adenovirus fibre according to Claim 1 or 2, **characterized in that** said adenovirus is the type 2 adenovirus (Ad2) and **in that** said fibre is modified by mutation consisting of substitution of one or more residues of the region between residues 441 to 478 and, preferably, 451 to 466 of said fibre.

8. Adenovirus fibre according to Claim 1, **characterized in that** said adenovirus is the type 2 adenovirus (Ad2) and **in that** said fibre is modified by mutation consisting of substitution of one or more residues of the region between residues 479 to 486 of said fibre.

9. Adenovirus fibre according to one of Claims 1 to 8, **characterized in that** it has a substantially reduced capacity for binding to the natural cellular receptor compared to an unmodified fibre and **in that** it is capable of trimerizing.

10. Adenovirus fibre according to one of Claims 1 to 9, **characterized in that** it comprises, in addition, a ligand capable of recognizing a cell surface molecule different from the natural cellular receptor of said adenovirus.

11. Adenovirus fibre according to Claim 10, **characterized in that** the ligand is selected from the group consisting of an antibody, a peptide, a hormone, a polypeptide or a sugar.

12. Adenovirus fibre according to Claim 10 or 11, **characterized in that** the ligand is inserted at the C-terminal end of the fibre.

13. DNA fragment or expression vector encoding an adenovirus fibre according to one of Claims 1 to 12.

14. Cell line **characterized in that** it comprises either in a form integrated into the genome or in the form of an episome, a DNA fragment according to Claim 13 placed under the control of the elements allowing its expression in said cell line.

15. Cell line according to Claim 14, **characterized in that** it is, in addition, capable of complementing an adenovirus deficient for one or more functions selected from the functions encoded by the E1, E2, E4 and L1-L5 regions.

16. Cell line according to Claim 14 or 15, **characterized in that** it is derived from the 293 line.

17. Adenovirus **characterized in that** it lacks a functional native fibre and **in that** it comprises a fibre according to one of Claims 1 to 12, said fibre according to one of Claims 1 to 12 being encoded by the genome of said adenovirus or provided *in trans* by a cell line according to one of Claims 14 to 16.

18. Adenovirus **characterized in that** it lacks a functional fibre and **in that** it comprises a fibre according to one of Claims 1 to 12 and a ligand capable of recognizing a cell surface molecule different from the natural cellular receptor of said adenovirus.

19. Adenovirus according to Claim 18, **characterized in that** the ligand is selected from the group consisting of an antibody, a peptide, a hormone, a polypeptide or a sugar.

20. Adenovirus according to either of Claims 18 and 19, **characterized in that** the ligand is inserted at the C-terminal end of the fibre.

21. Adenovirus according to one of Claims 17 to 19, **characterized in that** the ligand is inserted into a capsid protein other than the fibre, in particular the hexon or the penton.

22. Adenovirus according to one of Claims 17 to 21, **characterized in that** it is a replication-defective recombinant adenovirus.

23. Adenovirus according to Claim 22, **characterized in that** it is deleted for all or part of the E1 region and, optionally, for all or part of the E3 region.

24. Adenovirus according to Claim 23, **characterized in that** it is, in addition, deleted for all or part of the E2, E4 and/or L1-L5 region.

25. Adenovirus according to one of Claims 22 to 24, **characterized in that** it comprises a gene of interest selected from the genes encoding a cytokine, a cellular or nuclear receptor, a ligand, a coagulation factor, the CFTR protein, insulin, dystrophin, a growth hormone, an enzyme, an enzyme inhibitor, a polypeptide with antitumor effect, a polypeptide capable of inhibiting a bacterial, parasitic or viral infection and, in particular HIV, an antibody, a toxin, an immunotoxin and a marker.

26. Method of producing an adenovirus according to one of Claims 17 to 25, **characterized in that**:
- the genome of said adenovirus is transfected into an appropriate cell line,
- said transfected cell line is cultured under appropriate conditions in order to allow the production of said adenovirus, and
- said adenovirus is recovered from said culture of said transfected cell line and, optionally, said adenovirus is substantially purified.

27. Method for producing an adenovirus whose genome lacks all or part of the sequences encoding a fibre, **characterized in that**:
- the genome of said adenovirus is transfected into a cell line according to one of Claims 14 to 16,
- said transfected cell line is cultured under appropriate conditions in order to allow the production of said adenovirus, and
- said adenovirus is recovered from the culture of said transfected cell line and, optionally, said adenovirus is substantially purified.

28. Host cell comprising an adenovirus according to one of Claims 17 to 25 or obtained by a method according to Claim 26 or 27.

29. Pharmaceutical composition comprising an adenovirus according to one of Claims 17 to 25 or obtained by a method according to Claim 26 or 27 or a host cell according to Claim 28 in combination with a pharmaceutically acceptable carrier.

30. Therapeutic or prophylactic use of an adenovirus according to one of Claims 17 to 25 or obtained by a method according to Claim 26 or 27 or of a host cell according to Claim 28, for the preparation of a medicament intended for the treatment of the human or animal body by gene therapy.


### LISTE DE SEQUENCES

(1) INFORMATION GENERALE:
   (i) DEPOSANT:
      (A) NOM: Transgene S.A
      (B) RUE: 11 rue de Molsheim
      (C) VILLE: Strasbourg
      (E) PAYS: France
      (F) CODE POSTAL: 67000
      (G) TELEPHONE: (33) 03 88 27 91 00
      (H) TELECOPIE: (33) 03 88 27 91 11

      (A) NOM: Centre National de la Recherche Scientifique (CNRS)
      (B) RUE: 3 rue Michael Ange
      (C) VILLE: Paris
      (E) PAYS: France
      iF) CODE POSTAL: 75794 Cedex 16
      (G) TELEPHONE: (33) 01 44 96 40 00
      (H) TELECOPIE: (33) 01 44 96 50 00
   (ii) TITRE DE L' INVENTION: Fibre adenovirale modifiee et adenovirus cibles
   (iii) NOMBRE DE SEQUENCES: 40
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 581 acides aminés
      (B) TYPE: acide aminé
      (D) CONFIGURATION: linéaire
   (ii) TYPE DE MOLECULE: protéine
   (iii) HYPOTHETIQUE: NON
   (vi) ORIGINE:
      (A) ORGANISME: Mastadenovirus
      (B) SOUCHE: adenovirus 5
      (C) INDIVIDUEL ISOLE: fibre Ad5
   (viii) POSITION DANS LE GENOME:
      (B) POSITION SUR LA CARTE: 31063 a 33120 du genome Ad5
   (xi) DESCRIPTION DE LA SEQUENCE: SEQ ID NO: 1:
   Met Lys Arg Ala Arg Pro Ser Glu Asp Thr Phe Asn Pro Val Tyr Pro
   1               5                   10                  15
   Tyr Asp Thr Glu Thr Gly Pro Pro Thr Val Pro Phe Leu Thr Pro Pro
               20                  25                  30
   Phe Val Ser Pro Asn Gly Phe Gln Glu Ser Pro Pro Gly Val Leu Ser
           35                  40                  45
   Leu Arg Leu Ser Glu Pro Leu Val Thr Ser Asn Gly Met Leu Ala Leu
       50                  55                  60
   Lys Met Gly Asn Gly Leu Ser Leu Asp Glu Ala Gly Asn Leu Thr Ser
   65                  70                  75                  80
   Gln Asn Val Thr Thr Val Ser Pro Pro Leu Lys Lys Thr Lys Ser Asn
                   85                  90                  95
   Ile Asn Leu Glu Ile Ser Ala Pro Leu Thr Val Thr Ser Glu Ala Leu
               100                 105                 110
   Thr Val Ala Ala Ala Ala Pro Leu Met Val Ala Gly Asn Thr Leu Thr
           115                 120                 125
   Met Gln Ser Gln Ala Pro Leu Thr Val His Asp Ser Lys Leu Ser Ile
       130                 135                 140
   Ala Thr Gln Gly Pro Leu Thr Val Ser Glu Gly Lys Leu Ala Leu Gln
   145                 150                 155                 160
   Thr Ser Gly Pro Leu Thr Thr Thr Asp Ser Ser Thr Leu Thr Ile Thr
                   165                 170                 175
   Ala Ser Pro Pro Leu Thr Thr Ala Thr Gly Ser Leu Gly Ile Asp Leu
               180                 185                 190
   Lys Glu Pro Ile Tyr Thr Gln Asn Gly Lys Leu Gly Leu Lys Tyr Gly
           195                 200                 205
   Ala Pro Leu His Val Thr Asp Asp Leu Asn Thr Leu Thr Val Ala Thr
       210                 215                 220
   Gly Pro Gly Val Thr Ile Asn Asn Thr Ser Leu Gln Thr Lys Val Thr
   225                 230                 235                 240
   Gly Ala Leu Gly Phe Asp Ser Gln Gly Asn Met Gln Leu Asn Val Ala
                   245                 250                 255
   Gly Gly Leu Arg Ile Asp Ser Gln Asn Arg Arg Leu Ile Leu Asp Val
               260                 265                 270
   Ser Tyr Pro Phe Asp Ala Gln Asn Gln Leu Asn Leu Arg Leu Gly Gln
           275                 280                 285
   Gly Pro Leu Phe Ile Asn Ser Ala His Asn Leu Asp Ile Asn Tyr Asn
       290                 295                 300
   Lys Gly Leu Tyr Leu Phe Thr Ala Ser Asn Asn Ser Lys Lys Leu Glu
   305                 310                 315                 320
   Val Asn Leu Ser Thr Ala Lys Gly Leu Met Phe Asp Ala Thr Ala Ile
                   325                 330                 335
   Ala Ile Asn Ala Gly Asp Gly Leu Glu Phe Gly Ser Pro Asn Ala Pro
               340                 345                 350
   Asn Thr Asn Pro Leu Lys Thr Lys Ile Gly His Gly Leu Glu Phe Asp
           355                 360                 365
   Ser Asn Lys Ala Met Val Pro Lys Leu Gly Thr Gly Leu Ser Phe Asp
       370                 375                 380
   Ser Thr Gly Ala Ile Thr Val Gly Asn Lys Asn Asn Asp Lys Leu Thr
   385                 390                 395                 400
   Leu Trp Thr Thr Pro Ala Pro Ser Pro Asn Cys Arg Leu Asn Ala Glu
                   405                 410                 415
   Lys Asp Ala Lys Leu Thr Leu Val Leu Thr Lys Cys Gly Ser Gln Ile
               420                 425                 430
   Leu Ala Thr Val Ser Val Leu Ala Val Lys Gly Ser Leu Ala Pro Ile
           435                 440                 445
   Ser Gly Thr Val Gln Ser Ala His Leu Ile Ile Arg Phe Asp Glu Asn
       450                 455                 460
   Gly Val Leu Leu Asn Asn Ser Phe Leu Asp Pro Glu Tyr Trp Asn Phe
   465                 470                 475                 480
   Arg Asn Gly Asp Leu Thr Glu Gly Thr Ala Tyr Thr Asn Ala Val Gly
                   485                 490                 495
   Phe Met Pro Asn Leu Ser Ala Tyr Pro Lys Ser His Gly Lys Thr Ala
               500                 505                 510
   Lys Ser Asn Ile Val Ser Gln Val Tyr Leu Asn Gly Asp Lys Thr Lys
           515                 520                 525
   Pro Val Thr Leu Thr Ile Thr Leu Asn Gly Thr Gln Glu Thr Gly Asp
       530                 535                 540
   Thr Thr Pro Ser Ala Tyr Ser Met Ser Phe Ser Trp Asp Trp Ser Gly
   545                 550                 555                 560
   His Asn Tyr Ile Asn Glu Ile Phe Ala Thr Ser Ser Tyr Thr Phe Ser
                   565                 570                 575
   Tyr Ile Ala Gln Glu
               580
(2) INFORMATION POUR LA SEQ ID NO: 2:
   (i) CARACTERISTIQUES DE LA SEQUENCE:
      (A) LONGUEUR: 60 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de synthese oTG7000 (code pour PSASASASAPGS)
   (xi) DESCRIPTION DE LA SEQUENCE: SEQ ID NO: 2:
   AACGATTCTT TAGCTGCCGG GAGCAGAGGC GGAGGCGGAG GCGCTGGGTT CTTGGGCAAT 60
(2) INFORMATION POUR LA SEQ ID NO: 3:
   (i) CARACTERISTIQUES DE LA SEQUENCE:
      (A) LONGUEUR: 57 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de synthese otg7001 (code pour GRP)
   (xi) DESCRIPTION DE LA SEQUENCE: SEQ ID NO: 3:
   AACGATTCTT TACATCAGGT GGCCCACAGC CCAGTGGTTT CCGCTGCCGG GAGCAGA 57
(2) INFORMATION POUR LA SEQ ID NO: 4:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oliconucieotide de synthese oTG10776
   (xi) DESCRIPTION DE LA SEQUENCE: SEQ ID NO: 4:
   CCTTCCACGG GAAGATTGTA 20
(2) INFORMATION POUR LA SEQ ID NO: 5:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de synthese oTG10781
   (xi) DESCRIPTION DE LA SEQUENCE: SEQ ID NO: 5:
   GGGGTGTCTG TCTTCACACT 20
(2) INFORMATION POUR LA SEQ ID NO: 6:
   (i) CARACTERISTIQUES DE LA SEQUENCE:
      (A) LONGUEUR: 26 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de synthese oTG11065
   (xi) DESCRIPTION DE LA SEQUENCE: SEQ ID NO: 6:
   GGGAAGCTTG AGGTTAACCT AAGCAC 26
(2) INFORMATION POUR LA SEQ ID NO: 7:
   (i) CARACTERISTIQUES DE LA SEQUENCE:
      (A) LONGUEUR: 28 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleoticie de synthese oTG11066
   (xi) DESCRIPTION DE LA SEQUENCE: SEQ ID NO: 7:
   GGGTCTAGAG CTGCCGGGAG CAGAGGCG 28
(2) INFORMATION POUR LA SEQ ID NO: 8:
   (i) CARACTERISTIQUES DE LA SEQUENCE:
      (A) LONGUEUR: 29 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de synthese oTG11067
   (xi) DESCRIPTION DE LA SEQUENCE: SEQ ID NO: 8:
   GGGCTCGAGT TATGTTTCAA CGTGTTTAT 29
(2) INFORMATION POUR LA SEQ ID NO: 9:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de synthese oTG11068
   (xi) DESCRIPTION DE LA SEQUENCE: SEQ ID NO: 9:
   GTGCCCGGGG AGTTTATTAA TATC 24
(2) INFORMATION POUR LA SEQ ID NO: 10:
   (i) CARACTERISTIQUES DE LA SEQUENCE:
      (A) LONGUEUR: 31 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Homo sapiens
      (C) INDIVIDUEL ISOLE: oligonucleotide de synthese oTG11069 (clonage EGF)
   (xi) DESCRIPTION DE LA SEQUENCE: SEQ ID NO: 10:
   GCGTCTAGAA ATAGTGACTC TGAATGTCCC C 31
(2) INFORMATION POUR LA SEQ ID NO: 11:
   (i) CARACTERISTIQUES DE LA SEQUENCE:
      (A) LONGUEUR: 46 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (A) ORGANISME: Homo sapiens
      (C) INDIVIDUEL ISOLE: oligonucleotide de synthese oTG11070 (clonage EGF)
   (xi) DESCRIPTION DE LA SEQUENCE: SEQ ID NO: 11:
   GCGCTCGAGC ACAAACGATT CTTTAGCGCA GTTCCCACCA CTTCAG 46
(2) INFORMATION POUR LA SEQ ID NO: 12:
   (i) CARACTERISTIQUES DE LA SEQUENCE:
      (A) LONGUEUR: 42 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: adenovirus 5
      (C) INDIVIDUEL ISOLE: oligonucleotide de synthese oTG7414 (celecion du feuillet D)
   (xi) DESCRIPTION DE LA SEQUENCE: SEQ ID NO: 12:
   TAGCACTCCA TTTTCGTCGG ATCCTTGAAC TGTTCCAGAT AT 42
(2) INFORMATION POUR LA SEQ ID NO: 13:
   (i) CARACTERISTIQUES DE LA SEQUENCE:
      (A) LONGUEUR: 43 paires de bases
      (5) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: adenovirus 5 (Ad5)
      (C) INDIVIDUEL ISOLE: oligonucleotide de synthese oTGA (deletion de la boucle CD)
   (xi) DESCRIPTION DE LA SEQUENCE: SEQ ID NO: 13:
   CTTATAATAA GATGAGCACT GGATCCAGCC AAAACTGAAA CTG 43
(2) INFORMATION POUR LA SEQ ID NO: 14:
   (i) CARACTERISTIQUES DE LA SEQUENCE:
      (A) LONGUEUR: 45 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: adenovirus 5 (Ad5)
      (C) INDIVIDUEL ISOLE: oligonucleotide de synthese oTGB (deletion boucle CD et f.D)
   (xi) DESCRIPTION DE LA SEQUENCE: SEQ ID NO: 14:
   GTAGCACTCC ATTTTCGTCG GATCCAACAG CCAAAACTGA AACTG 45
(2) INFORMATION POUR LA SEQ ID NO: 15:
   (i) CARACTERISTIQUES DE LA SEQUENCE:
      (A) LONGUEUR: 88 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus humain Ad5 et Ad3
      (C) INDIVIDUEL ISOLE: oligonucieotide de synthese oTG11135 (boucle CD5 en CD3)
   (xi) DESCRIPTION DE LA SEQUENCE: SEQ ID NO: 15:
   CGTCAAATCT TATAATAAGA TGAGCACTCA CGTTTTTGTT TTTAAACAGG GTGTTGTAGT 60
   CGCTAACAGC CAAAACTGAA ACTGTAGC 88
(2) INFORMATION POUR LA SEQ ID NO: 16:
   (i) CARACTERISTIQUES DE LA SEQUENCE:
      (A) LONGUEUR: 64 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Adenovirus (Mastadenovirus)
      (B) SOUCHE: Adenovirus 3
      (C) INDIVIDUEL ISOLE: oligonucleotide de synthese oTG10350 (feuillet D5 en D3)
   (xi) DESCRIPTION DE LA SEQUENCE: SEQ ID NO: 16:
   GTAGCACTCC ATTTTCGTCA AAGTAGAGCT CCACGTTGAT ACTTTGAACT GTTCCAGATA      60
   TTGG                                                                   64
(2) INFORMATION POUR LA SEQ ID NO: 17:
   (i) CARACTERISTIQUES DE LA SEQUENCE:
      (A) LONGUEUR: 88 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus Ad5 et Ad3
      (C) INDIVIDUEL ISOLE: oligonucleotide de synthese oTG11136 (CD+D5 en CD+D3)
   (xi) DESCRIPTION DE LA SEQUENCE: SEQ ID NO: 17:
   CGTCAAAGTA GAGCTCCACG TTGATACTCA CGTTTTTGTT TTTAAACAGG GTGTTGTAGT      60
   CGCTAACAGC CAAAACTGAA ACTGTAGC                                         88
(2) INFORMATION POUR LA SEQ ID NO: 18:
   (i) CARACTERISTIQUES DE LA SEQUENCE:
      (A) LONGUEUR: 56 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5
      (C) INDIVIDUEL ISOLE: oligonucleotide de synthese oTGC (rempl. coude GSLR en DKLT)
   (xi) DESCRIPTION DE LA SEQUENCE: SEQ ID NO: 18:
   TTGAACTGTT CCAGATATTG GGGTCAGTTT GTCTTTAACA GCCAAAACTG AAACTG 56
(2) INFORMATION POUR LA SEQ ID NO: 19:
   (i) CARACTERISTIQUES DE LA SEQUENCE:
      (A) LONGUEUR: 48 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTGD (rempl. coude SGTV en DKLT)
   (xi) DESCRIPTION DE LA SEQUENCE: SEQ ID NO: 19:
   AATAAGATGA GCACTTTGGG TCAGTTTGTC TATTGGAGCC AAACTGCC 48
(2) INFORMATION POUR LA SEQ ID NO: 20:
   (i) CARACTERISTIQUES DE LA SEQUENCE:
      (A) LONGUEUR: 44 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5
      (C) INDIVIDUEL ISOLE: oligonucleotide de synthese oTGE (rempl. G443 en D)
   (xi) DESCRIPTION DE LA SEQUENCE: SEQ ID NO: 20:
   CCAGATATTG GAGCCAAACT GTCTTTAACA GCCAAAACTG AAAC 44
(2) INFORMATION POUR LA SEQ ID NO: 21:
   (i) CARACTERISTIQUES DE LA SEQUENCE:
      (A) LONGUEUR: 42 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTGF (rempl. L445 en F)
   (xi) DESCRIPTION DE LA SEQUENCE: SEQ ID NO: 21:
   TGTTCCAGAT ATTGGAGCGA AACTGCCTTT AACAGCCAAA AC 42
(2) INFORMATION POUR LA SEQ ID NO: 22:
   (i) CARACTERISTIQUES DE LA SEQUENCE:
      (A) LONGUEUR: 42 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTGG (rempl. G450 en N)
   (xi) DESCRIPTION DE LA SEQUENCE: SEQ ID NO: 22:
   ATGAGCACTT TGAACTGTGT TAGATATTGG AGCCAAACTG CC 42
(2) INFORMATION POUR LA SEQ ID NO: 23:
   (i) CARACTERISTIQUES DE LA SEQUENCE:
      (A) LONGUEUR: 44 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTGH (rempl. T451 en K)
   (xi) DESCRIPTION DE LA SEQUENCE: SEQ ID NO: 23:
   TAAGATGAGC ACTTTGAACC TTTCCAGATA TTGGAGCCAA ACTG 44
(2) INFORMATION POUR LA SEQ ID NO: 24:
   (i) CARACTERISTIQUES DE LA SEQUENCE:
      (A) LONGUEUR: 45 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTGI (rempl. V452 en N)
   (xi) DESCRIPTION DE LA SEQUENCE: SEQ ID NO: 24:
   CTTATAATAA GATGAGCACT TTGGTTTGTT CCAGATATTG GAGCC 45
(2) INFORMATION POUR LA SEQ ID NO: 25:
   (i) CARACTERISTIQUES DE LA SEQUENCE:
      (A) LONGUEUR: 48 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5
      (C) INDIVIDUEL ISOLE: oligonucleotide de synthese oTGJ (rempl. A455 en F)
   (xi) DESCRIPTION DE LA SEQUENCE: SEQ ID NO: 25:
   GTCAAATCTT ATAATAAGAT GGAAACTTTG AACTGTTCCA GATATTGG 48
(2) INFORMATION POUR LA SEQ ID NO: 26:
   (i) CARACTERISTIQUES DE LA SEQUENCE:
      (A) LONGUEUR: 47 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5
      (C) INDIVIDUEL ISOLE: oligonucleotide de synthese oTGK (rempl. L457 en K)
   (xi) DESCRIPTION DE LA SEQUENCE: SEQ ID NO: 26:
   CCATTTTCGT CAAATCTTAT AATTTTATGA GCACTTTGAA CTGTTCC 47
(2) INFORMATION POUR LA SEQ ID NO: 27:
   (i) CARACTERISTIQUES DE LA SEQUENCE:
      (A) LONGUEUR: 46 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTGL (rempl.I459 en A)
   (xi) DESCRIPTION DE LA SEQUENCE: SEQ ID NO: 27:
   GCACTCCATT TTCGTCAAAT CTAGCAATAA GATGAGCACT TTGAAC 46
(2) INFORMATION POUR LA SEQ ID NO: 28:
   (i) CARACTERISTIQUES DE LA SEQUENCE:
      (A) LONGUEUR: 23 paires de bases
      (3) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Mastadenovirus
      (5) SOUCHE: Adenovirus 5 (Ad5)
      (C) INDIVIDUEL ISOLE: oligonucleotide de synthese oTG11102 (clonage hexon)
   (xi) DESCRIPTION DE LA SEQUENCE: SEQ ID NO: 23:
(2) INFORMATION POUR LA SEQ ID NO: 29:
   (i) CARACTERISTIQUES DE LA SEQUENCE:
      (A) LONGUEUR: 38 paires de bases
      (3) TYPE: acide nucléique
      (C) NOMBRE DE BRINS: simple
      (D) CONFIGURATION: linéaire
   (ii) TYPE DE MOLECULE: ADN (gènomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTG11103 (clonage hexon)
   (xi) DESCRIPTION DE LA SEQUENCE: SEQ ID NO: 29:
   GGCCTCTAGA GTTGAGAAAA ATTGCATTTC CACTTGAC 38
(2) INFORMATION POUR LA SEQ ID NO: 30:
   (i) CARACTERISTIQUES DE LA SEQUENCE:
      (A) LONGUEUR: 23 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTG11104 (clonage hexon)
   (xi) DESCRIPTION DE LA SEQUENCE: SEQ ID NO: 30:
   GGTATTGTAC AGTGAAGATG TAG 23
(2) INFORMATION POUR LA SEQ ID NO: 31:
   (i) CARACTERISTIQUES DE LA SEQUENCE:
      (A) LONGUEUR: 23 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTG11105
   (xi) DESCRIPTION DE LA SEQUENCE: SEQ ID NO: 31:
   CGTTGGAAGG ACTGTACTTT AGC 23
(2) INFORMATION POUR LA SEQ ID NO: 32:
   (i) CARACTERISTIQUES DE LA SEQUENCE:
      (A) LONGUEUR: 38 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Homo sapiens
      (C) INDIVIDUEL ISOLE: oligonucleotide de synthese oTG11106 (clonage cDNA EGF)
   (xi) DESCRIPTION DE LA SEQUENCE: SEQ ID NO: 32:
   CGCGTCTAGA GGCGAATAGT GACTCTGAAT GTCCCCTG 38
(2) INFORMATION POUR LA SEQ ID NO: 33:
   (i) CARACTERISTIQUES DE LA SEQUENCE:
      (A) LONGUEUR: 45 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (vi) ORIGINE:
      (A) ORGANISME: Homo sapiens
      (C) INDIVIDUEL ISOLE: oligonucleotide de synthese oTG11107 (clonage cDNA EGF)
   (xi) DESCRIPTION DE LA SEQUENCE: SEQ ID NO: 33:
   CCACTGTACA ATACCACTTT AGGGCGCAGT TCCCACCACT TCAGG 45
(2) INFORMATION POUR LA SEQ ID NO: 34:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTG7171 (deletion de la fibre)
   (xi) DESCRIPTION DE LA SEQUENCE: SEQ ID NO: 34:
   ATGGTTAACT TGCACCAGTG C 21
(2) INFORMATION POUR LA SEQ ID NO: 35:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTG7275 (deletion de la fibre)
   (xi) DESCRIPTION DE LA SEQUENCE: SEQ ID NO: 35:
   GGGCTCGAGC TGCAACAACA TGAAGAT 27
(2) INFORMATION POUR LA SEQ ID NO: 36:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTG7276 (deletion de la fibre)
   (xi) DESCRIPTION DE LA SEQUENCE: SEQ ID NO: 36:
   CCGCTCGAGA CTCCTCCCTT TGTATCC 27
(2) INFORMATION POUR LA SEQ ID NO: 37:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 5 (Ad5)
      (C) INDIVIDUEL ISOLE: oligonucleotide de synthese oTG7049 (deletion de la fibre)
   (xi) DESCRIPTION DE LA SEQUENCE: SEQ ID NO: 37:
   CTGCCCGGGA GTTTATTAAT 20
(2) INFORMATION POUR LA SEQ ID NO: 38:
   (i) CARACTERISTIQUES DE LA SEQUENCE:
      (A) LONGUEUR: 42 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTSETIQUE: NON
   (iii) ANTI-SENS: OUI
   (vi) ORIGINE:
      (A) ORGANISME: Mastadenovirus
      (B) SOUCHE: adenovirus 5
      (C) INDIVIDUEL ISOLE: oligonucleotide de synthese oTG7416 (deletion du feuillet H)
   (xi) DESCRIPTION DE LA SEQUENCE: SEQ ID NO: 38:
   TGTTTCCTGT GTACCGTTGG ATCCTTTAGT TTTGTCTCCG TT 42
(2) INFORMATION POUR LA SEQ ID NO: 39:
   (i) CARACTERISTIQUES DE LA SEQUENCE:
      (A) LONGUEUR: 64 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: Adenovirus 3
      (C) INDIVIDUEL ISOLE: oligonucleotide de synthese oTG10352 (feuillet H5 en H3)
   (xi) DESCRIPTION DE LA SEQUENCE: SEQ ID NO: 39:
   TGTTTCCTGT GTACCGTTTA GCATCACGGT CACCTCGAGA GGTTTAGTTT TGTCTCCGTT 60
   TAAG 64
(2) INFORMATION POUR LA SEQ ID NO: 40:
   (i) CARACTERISTIQUES DE LA SEQUENCE:
      (A) LONGUEUR: 42 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Mastadenovirus
      (B) SOUCHE: adenovirus 5
      (C) INDIVIDUEL ISOLE: oligonucleotide de synthese (deletion feuillets E et F)
   (xi) DESCRIPTION DE LA SEQUENCE: SEQ ID NO: 40:
   TGTATAGGCT GTGCCTTCGG ATCCCCAATA TTCTGGGTCC AG                    42